# EUROPEAN PATENT SPECIFICATION

(11) **EP 3 644 122 B1**
(45) Date of publication and mention of the grant of the patent: **30.12.2020**
(21) Application number: 19202616.9
(22) Date of filing: 11.10.2019
(51) Int. Cl.: G03F 7/004, G03F 7/038, C07C 311/01, C07C 311/02

(54) **NEGATIVE RESIST COMPOSITION AND RESIST PATTERN FORMING PROCESS**
NEGATIVE RESISTZUSAMMENSETZUNG UND RESISTMUSTERFORMUNGSVORGANG
COMPOSITION DE RÉSERVE NÉGATIVE ET PROCÉDÉ DE FORMATION DE MOTIFS DE RÉSERVE

(30) Priority: 25.10.2018 JP 2018200797
(43) Date of publication of application: 29.04.2020
(73) Proprietor: Shin-Etsu Chemical Co., Ltd., Tokyo (JP)
(72) Inventor: DOMON, Daisuke, Joetsu-shi, Niigata (JP); INOUE, Naoya, Joetsu-shi, Niigata (JP); OHASHI, Masaki, Joetsu-shi, Niigata (JP); MASUNAGA, Keiichi, Joetsu-shi, Niigata (JP); KOTAKE, Masaaki, Joetsu-shi, Niigata (JP)
(74) Representative: Ter Meer Steinmeister & Partner

(56) References cited:
- US-A1- 2011 008 731
- US-A1- 2015 125 794
- US-A1- 2017 184 963

## Description

### TECHNICAL FIELD

This invention relates to an onium salt, a negative resist composition, and a resist pattern forming process using the same.

### BACKGROUND ART

To meet the recent demand for higher integration in integrated circuits, pattern formation to a finer feature size is required. Acid-catalyzed chemically amplified resist compositions are most often used in forming resist patterns with a feature size of 0.2 µm or less. High-energy radiation such as UV, deep-UV or EB is used as the light source for exposure of these resist compositions. In particular, while EB lithography is utilized as the ultra-fine microfabrication technique, it is also indispensable in processing a photomask blank to form a photomask for use in semiconductor device fabrication.

Polymers comprising a major proportion of aromatic structure having an acidic side chain, for example, polyhydroxystyrene are useful in resist materials for the KrF excimer laser lithography. These polymers are not used in resist materials for the ArF excimer laser lithography since they exhibit strong absorption at a wavelength of around 200 nm. These polymers, however, are expected to form useful resist materials for the EB and EUV lithography for forming patterns of finer size than the processing limit of ArF excimer laser because they offer high etching resistance.

Resist compositions for photolithography include positive ones in which exposed areas are dissolved away and negative ones in which exposed areas are left as a pattern. A viable composition is selected among them depending on the desired resist pattern. In general, the chemically amplified negative resist composition comprises a polymer which is normally soluble in an aqueous alkaline developer, an acid generator which is decomposed to generate an acid upon exposure to light, and a crosslinker which causes the polymer to crosslink in the presence of the acid serving as a catalyst, thus rendering the polymer insoluble in the developer (sometimes, the crosslinker is incorporated in the polymer). Typically a quencher is added for controlling the diffusion of the acid generated upon light exposure.

Typical of the alkali-soluble units to constitute polymers which dissolve in aqueous alkaline developer are units derived from phenols. A number of negative resist compositions of such type were developed, especially as adapted for exposure to KrF excimer laser light. These compositions have not been used in the ArF excimer laser lithography because the phenolic units are not transmissive to exposure light having a wavelength of 150 to 220 nm. Recently, these compositions are recognized attractive again as the negative resist composition for the short wavelength (e.g., EB or EUV) lithography capable of forming finer size patterns. Exemplary compositions are described in Patent Documents 1 to 3.

Improvements were made in the control of resist sensitivity and pattern profile by properly selecting and combining components used in resist compositions and adjusting processing conditions. One outstanding problem is the diffusion of acid that has a significant impact on the resolution of a chemically amplified resist composition.

A quencher is, in fact, essential for controlling acid diffusion and improving the performance, especially resolution of a resist composition. Studies have been made on the quencher while amines and weak acid onium salts have been generally used. The weak acid onium salts are exemplified in several patent documents. Patent Document 4 describes that the addition of triphenylsulfonium acetate ensures to form a satisfactory resist pattern without T-top profile, a difference in line width between isolated and grouped patterns, and standing waves. Patent Document 5 describes improvements in sensitivity, resolution and exposure margin by the addition of sulfonic acid ammonium salts or carboxylic acid ammonium salts. Also, Patent Document 6 describes that a resist composition for KrF or EB lithography comprising a PAG capable of generating a fluorinated carboxylic acid is improved in resolution and process latitude such as exposure margin and depth of focus. Further, Patent Document 7 describes that a resist composition for F₂ laser lithography comprising a PAG capable of generating a fluorinated carboxylic acid is improved in line edge roughness (LER) and solves the footing problem. Patent Documents 4 to 7 refer to the KrF, EB and F₂ lithography.

Patent Document 8 describes a positive photosensitive composition for ArF excimer laser lithography comprising a carboxylic acid onium salt. These systems are based on the mechanism that a salt exchange occurs between a weak acid onium salt and a strong acid (sulfonic acid) generated by a PAG upon exposure, to form a weak acid and a strong acid onium salt. That is, the strong acid (sulfonic acid) having high acidity is replaced by a weak acid (carboxylic acid), thereby suppressing acid-catalyzed decomposition reaction of acid labile group and reducing or controlling the distance of acid diffusion. The onium salt apparently functions as a quencher.
US 2011/008731 A1 relates to an actinic-ray- or radiation-sensitive resin composition including a compound (A) being an onium salt that when exposed to actinic rays or radiation, generates an acid and a resin (B) whose rate of dissolution into an alkali developer is increased by the action of an acid.

However, when a resist composition comprising the foregoing carboxylic acid onium salt or fluorocarboxylic acid onium salt is used in patterning, LER still remains as an outstanding problem. It would be desirable to have a quencher capable of minimizing LER.

### Citation List

Patent Document 1: JP-A 2006-201532 (US 20060166133, EP 1684118)
Patent Document 2: JP-A 2006-215180
Patent Document 3: JP-A 2008-249762 (USP 9075306, EP 1975711)
Patent Document 4: JP 3955384 (USP 6479210)
Patent Document 5: JP-A H11-327143
Patent Document 6: JP 4231622 (USP 6485883)
Patent Document 7: JP 4116340 (USP 7214467)
Patent Document 8: JP 4226803 (USP 6492091)
Patent Document 9: JP-A 2001-330947 (USP 6605409, EP 1158363)

### DISCLOSURE OF INVENTION

An object of the invention is to provide a negative resist composition comprising a specific onium salt as quencher, which is processed by lithography to form a resist pattern with improved resolution and minimal LER, and a pattern forming process using the resist composition.

The inventors have found that a resist composition comprising an onium salt of benzenesulfonimide having a bulky substituent group or sulfonimide having an alicyclic structure as a quencher can be processed by lithography to form a resist pattern with minimal LER.
In an aspect, the invention relates to the subject-matter as defined by claim 1. In a preferred embodiment, the polymer further comprises recurring units of at least one type selected from recurring units having the formulae (B2), (B3) and (B4). 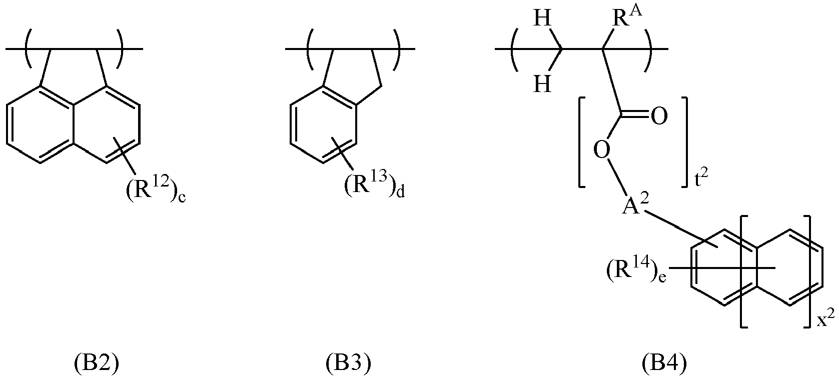 Herein R^{A} is hydrogen, fluorine, methyl or trifluoromethyl; R¹² and R¹³ are each independently hydroxyl, halogen, acetoxy, an optionally halogenated C₁-C₈ alkyl group, optionally halogenated C₁-C₈ primary alkoxy group, C₂-C₈ secondary alkoxy group, optionally halogenated C₂-C₈ acyloxy group, or optionally halogenated C₂-C₈ alkylcarbonyloxy group; R¹⁴ is an acetyl group, acetoxy group, C₁-C₂₀ alkyl group, C₁-C₂₀ primary alkoxy group, C₂-C₂₀ secondary alkoxy group, C₂-C₂₀ acyloxy group, C₂-C₂₀ alkoxyalkyl group, C₂-C₂₀ alkylthioalkyl group, halogen atom, nitro group, or cyano group; A² is a single bond or C₁-C₁₀ alkanediyl group in which an ether bond may intervene in a carbon-carbon bond; c and d are each independently an integer of 0 to 4, e is an integer of 0 to 5, x² is an integer of 0 to 2, and t² is 0 or 1.

In a preferred embodiment, the polymer further comprises recurring units having the formula (B5). 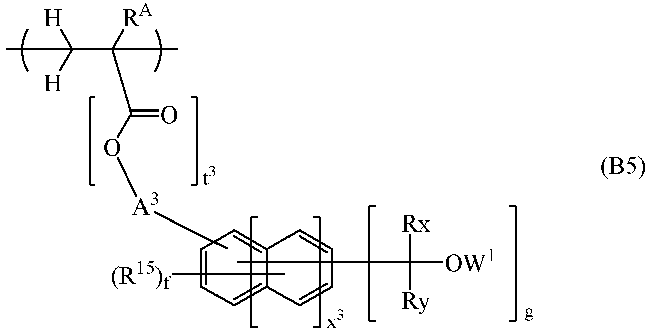 Herein R^{A} is hydrogen, fluorine, methyl or trifluoromethyl; R¹⁵ is each independently halogen, an optionally halogenated C₂-C₈ acyloxy group, optionally halogenated C₁-C₆ alkyl group, or optionally halogenated C₁-C₆ alkoxy group; A³ is a single bond or C₁-C₁₀ alkanediyl group in which an ether bond may intervene in a carbon-carbon bond; W¹ is hydrogen, a C₁-C₁₀ monovalent aliphatic hydrocarbon group in which an ether bond, carbonyl moiety or carbonyloxy moiety may intervene in a carbon-carbon bond, or an optionally substituted monovalent aromatic ring group; Rx and Ry are each independently hydrogen, an optionally hydroxy or alkoxy-substituted C₁-C₁₅ alkyl group or an optionally substituted monovalent aromatic ring group, with the proviso that both Rx and Ry are not hydrogen at the same time, Rx and Ry may bond together to form a ring with the carbon atom to which they are attached; x³ is an integer of 0 to 2, t³ is 0 or 1, f is an integer satisfying 0 ≤ f ≤ 5+2x³-g, and g is an integer of 1 to 3.

In a preferred embodiment, the polymer further comprises recurring units of at least one type selected from recurring units having the formulae (B6), (B7) and (B8). 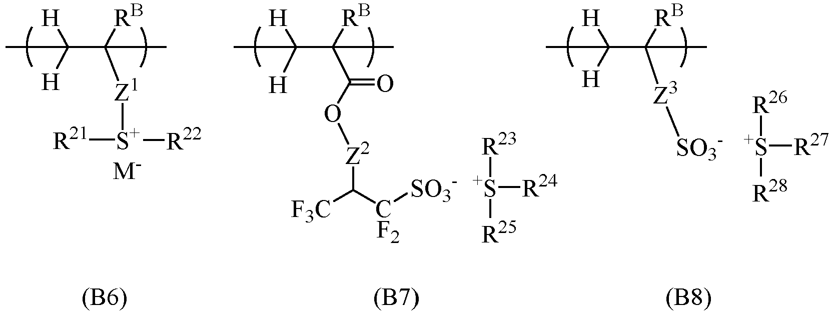 Herein R^{B} is each independently hydrogen or methyl; Z¹ is a single bond, phenylene group, -O-Z¹¹-, -C(=O)-O-Z¹¹-, or -C(=O)-NH-Z¹¹-, Z¹¹ is a C₁-C₆ alkanediyl, C₂-C₆ alkenediyl or phenylene group, which may contain a carbonyl moiety, ester bond, ether bond or hydroxyl moiety; Z² is a single bond or -Z²¹-C(=O)-O-, Z²¹ is a C₁-C₂₀ divalent hydrocarbon group which may contain a heteroatom; Z³ is a single bond, methylene, ethylene, phenylene, fluorinated phenylene, -O-Z³¹-, -C(=O)-O-Z³¹-, or -C(=O)-NH-Z³¹-, Z³¹ is a C₁-C₆ alkanediyl, C₂-C₆ alkenediyl or phenylene group, which may contain a carbonyl moiety, ester bond, ether bond or hydroxyl moiety; R²¹ to R²⁸ are each independently a C₁-C₂₀ monovalent hydrocarbon group which may contain a heteroatom, or R²¹ and R²² may bond together to form a ring with the sulfur atom to which they are attached, or any two or more of R²³, R²⁴ and R²⁵ may bond together to form a ring with the sulfur atom to which they are attached, any two or more of R²⁶, R²⁷ and R²⁸ may bond together to form a ring with the sulfur atom to which they are attached; and M⁻ is a non-nucleophilic counter ion.

In a preferred embodiment, the polymer comprises recurring units having the formula (B1-1), recurring units having the formula (B5-1), and recurring units having the formula (B7). 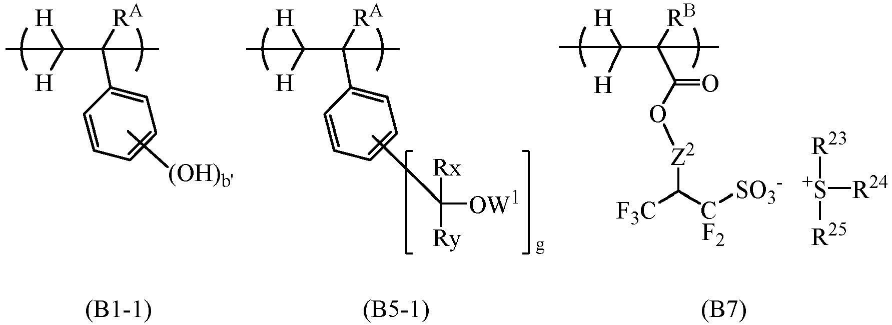 Herein R^{A} is each independently hydrogen, fluorine, methyl or trifluoromethyl; R^{B} is hydrogen or methyl; W¹ is hydrogen, a C₁-C₁₀ monovalent aliphatic hydrocarbon group in which an ether bond, carbonyl moiety or carbonyloxy moiety may intervene in a carbon-carbon bond, or an optionally substituted monovalent aromatic ring group; Rx and Ry are each independently hydrogen, an optionally hydroxy or alkoxy-substituted C₁-C₁₅ alkyl group or an optionally substituted monovalent aromatic ring group, with the proviso that both Rx and Ry are not hydrogen at the same time, Rx and Ry may bond together to form a ring with the carbon atom to which they are attached; Z² is a single bond or -Z²¹-C(=O)-O-, Z²¹ is a C₁-C₂₀ divalent hydrocarbon group which may contain a heteroatom; R²³ to R²⁵ are each independently a C₁-C₂₀ monovalent hydrocarbon group which may contain a heteroatom, or any two or more of R²³, R²⁴ and R²⁵ may bond together to form a ring with the sulfur atom to which they are attached; b' is an integer of 1 to 3, and g is an integer of 1 to 3.

In a preferred embodiment, the base polymer (B) further contains a polymer free of recurring units having the formula (B6), recurring units having the formula (B7), and recurring units having the formula (B8).

The negative resist composition may further comprise (C) a crosslinker.

In another embodiment, the negative resist composition is free of a crosslinker.

The negative resist composition may further comprise (E) an acid generator.

In a further aspect, the invention provides a photomask blank coated with the negative resist composition defined above.

In a still further aspect, the invention provides a resist pattern forming process comprising the steps of applying the negative resist composition defined above onto a substrate to form a resist film thereon, exposing the resist film patternwise to high-energy radiation, and developing the resist film in an alkaline developer to form a resist pattern.

Preferably, the high-energy radiation is EUV or EB.

In one embodiment, the substrate is a photomask blank.

### ADVANTAGEOUS EFFECTS OF INVENTION

Owing to the onium salt, the negative resist composition of the invention is effective for controlling acid diffusion during the exposure step. When the composition is coated as a resist film and processed to form a pattern, the resist film exhibits a very high resolution during pattern formation, and forms a pattern with minimal LER. The pattern forming process using the negative resist composition can form a resist pattern with minimal LER while maintaining a high resolution. The invention is best suited for a micropatterning process, typically EUV or EB lithography.

### DESCRIPTION OF EMBODIMENTS

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group. In chemical formulae, Me stands for methyl, Ac stands for acetyl, and the broken line designates a valence bond.

The abbreviations and acronyms have the following meaning.
EB: electron beam
EUV: extreme ultraviolet
Mw: weight average molecular weight
Mn: number average molecular weight
Mw/Mn: molecular weight distribution or dispersity
GPC: gel permeation chromatography
PEB: post-exposure bake
PAG: photoacid generator
LER: line edge roughness

It is understood that for some structures represented by chemical formulae, there can exist enantiomers and diastereomers because of the presence of asymmetric carbon atoms. In such a case, a single formula collectively represents all such isomers. The isomers may be used alone or in admixture.

### Onium salt

The onium salt present in the negative resist composition has the formula (A).

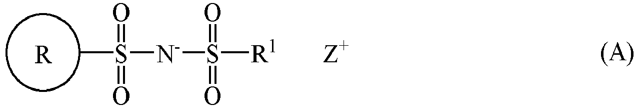

In formula (A), R¹ is a C₁-C₂₀ monovalent hydrocarbon group which may contain a heteroatom. The monovalent hydrocarbon group may be straight, branched or cyclic. In the hydrocarbon group, some hydrogen may be substituted by a moiety containing a heteroatom such as oxygen, sulfur, nitrogen or halogen, or some carbon may be replaced by a moiety containing a heteroatom such as oxygen, sulfur or nitrogen, so that the group may contain a hydroxyl moiety, cyano moiety, carbonyl moiety, ether bond, thioether bond, ester bond, sulfonic acid ester bond, carbonate bond, carbamate bond, lactone ring, sultone ring, carboxylic anhydride (-C(=O)-O-C(=O)-) or haloalkyl moiety.

Examples of the C₁-C₂₀ monovalent hydrocarbon group which may contain a heteroatom include C₁-C₂₀ straight or branched alkyl groups such as methyl, ethyl, propyl, isopropyl, n-butyl, sec-butyl, tert-butyl, n-pentyl, tert-pentyl, n-hexyl, n-octyl, n-nonyl, n-decyl, C₁-C₂₀ monovalent saturated cyclic hydrocarbon groups such as cyclopentyl, cyclohexyl, 2-ethylhexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decanyl, adamantyl, and adamantylmethyl, C₆-C₁₀ aryl groups such as phenyl, naphthyl, and anthracenyl, and halo-substituted alkyl groups such as fluoromethyl, difluoromethyl, trifluoromethyl, 2,2,2-trifluoroethyl, 1,1,2,2,2-pentafluoroethyl, and 1,1,1,3,3,3-hexafluoroethyl.

The ring R is a group having the formula (A1), or a C₆-C₃₀ group having an alicyclic structure in which some carbon may be replaced by a moiety containing sulfur, oxygen or nitrogen.

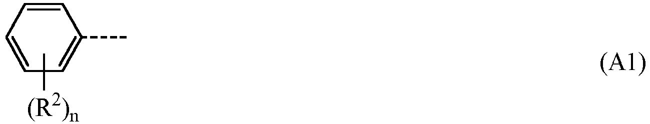

In formula (A1), the broken line designates a valence bond. R² is each independently a C₁-C₂₀ monovalent hydrocarbon group which may contain a heteroatom. The monovalent hydrocarbon group may be straight, branched or cyclic. In the hydrocarbon group, some hydrogen may be substituted by a moiety containing a heteroatom such as oxygen, sulfur, nitrogen or halogen, or some carbon may be replaced by a moiety containing a heteroatom such as oxygen, sulfur or nitrogen, so that the group may contain a hydroxyl moiety, cyano moiety, carbonyl moiety, ether bond, thioether bond, ester bond, sulfonic acid ester bond, carbonate bond, carbamate bond, lactone ring, sultone ring, carboxylic anhydride (-C(=O)-O-C(=O)-) or haloalkyl moiety.

Examples of the C₁-C₂₀ monovalent hydrocarbon group which may contain a heteroatom, represented by R², include C₁-C₂₀ straight or branched alkyl groups such as methyl, ethyl, propyl, isopropyl, n-butyl, sec-butyl, tert-butyl, n-pentyl, tert-pentyl, n-hexyl, n-octyl, n-nonyl, n-decyl, C₁-C₂₀ monovalent saturated cyclic hydrocarbon groups such as cyclopentyl, cyclohexyl, 2-ethylhexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decanyl, adamantyl, and adamantylmethyl, and C₆-C₁₀ aryl groups such as phenyl, naphthyl, and anthracenyl.

Of these, R² is preferably a C₃-C₂₀ branched or cyclic monovalent hydrocarbon group. This configuration ensures that the sulfonimide acid generated after neutralization has a fully bulky structure. Accordingly, when the sulfonimide acid generated after neutralization contributes slightly to insolubilization, its reactivity is suppressed, contributing to an improvement in LER.

In formula (A1), preferably at least one R² is bonded at the ortho-position relative to the carbon atom bonded to the sulfonyl group. With this configuration, the active site on the sulfonimide acid generated after neutralization is sterically blocked by the substituent group at ortho-position whereby reactivity is suppressed. Accordingly, when the sulfonimide acid generated after neutralization contributes slightly to insolubilization, reactivity is suppressed, contributing to an improvement in LER.

In formula (A1), n is an integer of 2 to 5, preferably 3 to 5.

Examples of the C₆-C₃₀ group having an alicyclic structure, represented by the ring R, are shown below, but not limited thereto.

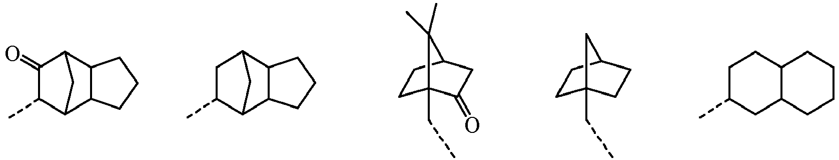

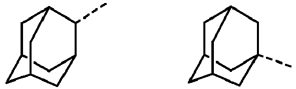

In formula (A), Z⁺ is a sulfonium cation having the formula (A2) or an iodonium cation having the formula (A3).

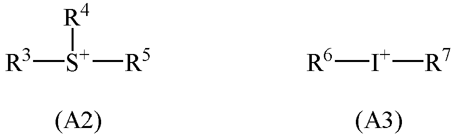

In formulae (A2) and (A3), R³ to R⁷ are each independently a C₁-C₂₀ monovalent hydrocarbon group which may contain a heteroatom, any two or more of R³, R⁴ and R⁵ may bond together to form a ring with the sulfur atom to which they are attached.

The C₁-C₂₀ monovalent hydrocarbon group which may contain a heteroatom, represented by R³ to R⁷, may be straight, branched or cyclic. Specific examples thereof are as exemplified above for R¹ in formula (A).

When any two or more of R³, R⁴ and R⁵ in formula (A2) bond together to form a ring with the sulfur atom to which they are attached, examples of the ring are shown below, but not limited thereto.

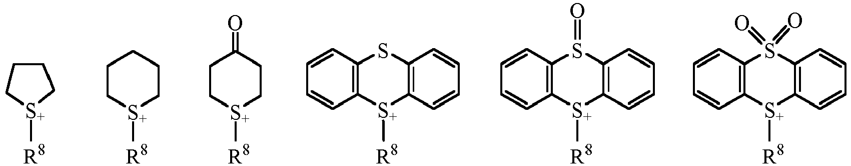

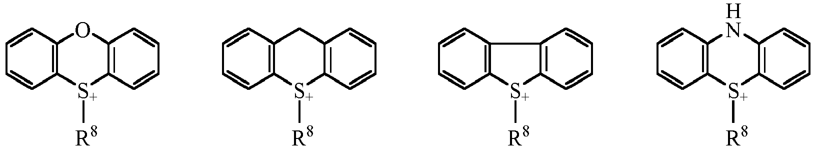

In the above formulae, R⁸ is a C₁-C₂₀ monovalent hydrocarbon group which may contain a heteroatom, examples of which are as exemplified above for R¹ in formula (A).

Examples of the sulfonium cation having formula (A2) are given below, but not limited thereto.

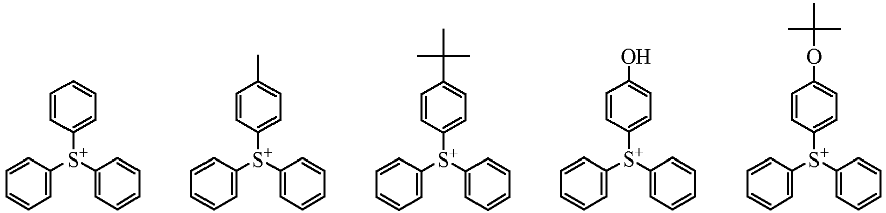

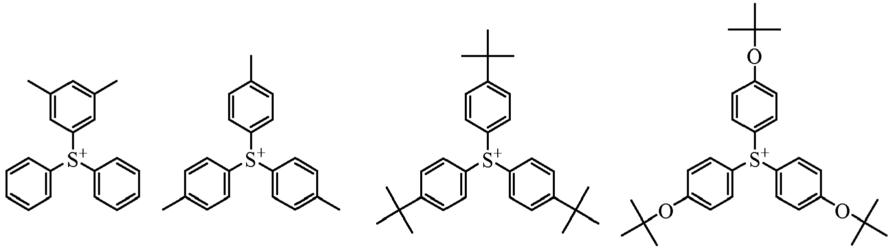

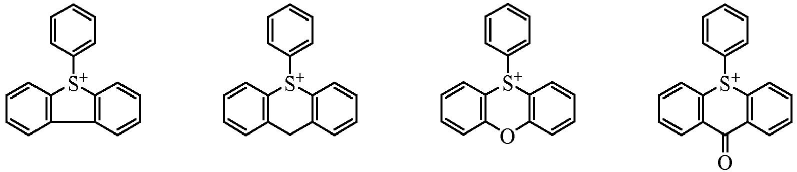

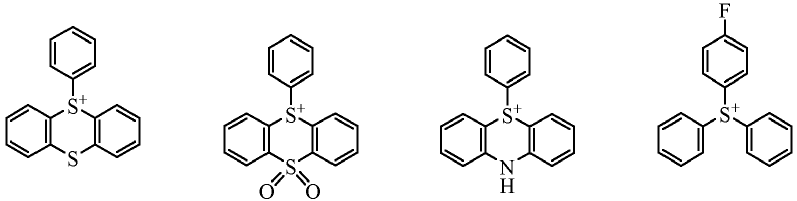

In formula (A3), the C₁-C₂₀ monovalent hydrocarbon group which may contain a heteroatom, represented by R⁶ and R⁷, is preferably an aryl group.

Preferred examples of the iodonium cation having formula (A3) include bis(4-methylphenyl)iodonium, bis(4-ethylphenyl)iodonium, bis(4-tert-butylphenyl)iodonium, bis[4-(1,1-dimethylpropyl)phenyl]iodonium, 4-methoxyphenylphenyliodonium, 4-tert-butoxyphenylphenyliodonium, 4-acryloyloxyphenylphenyliodonium, and 4-methacryloyloxyphenylphenyliodonium, with bis(4-tert-butylphenyl)iodonium being most preferred.

Examples of the anion in the onium salt having formula (A) are given below, but not limited thereto.

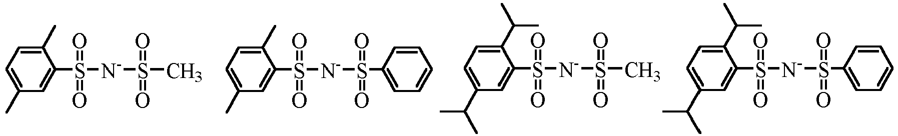

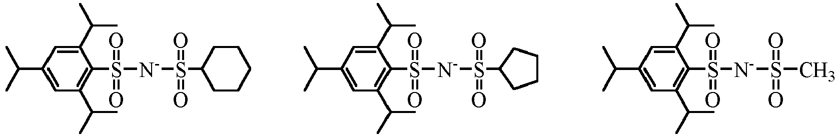

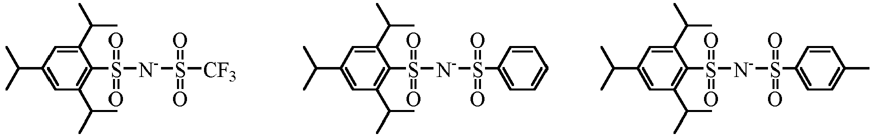

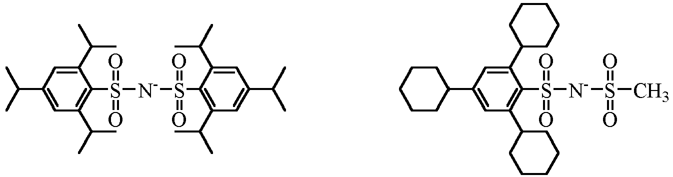

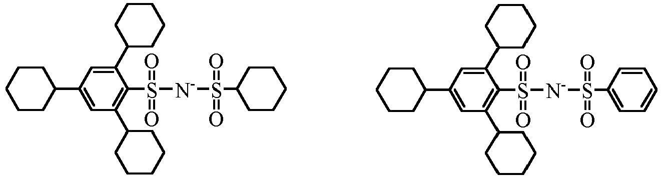

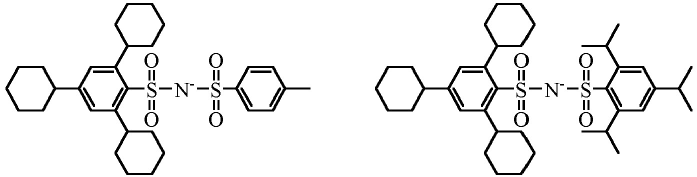

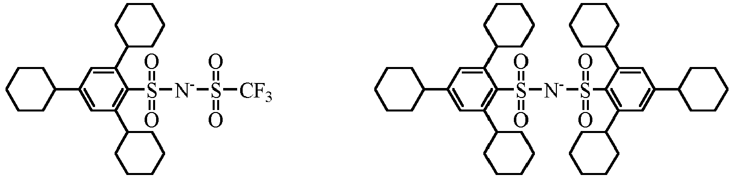

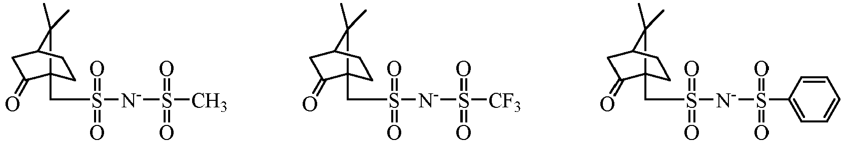

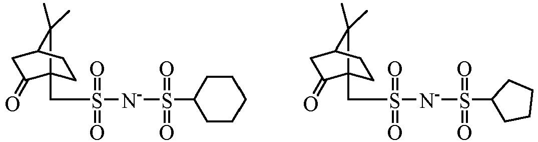

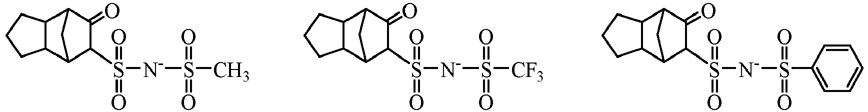

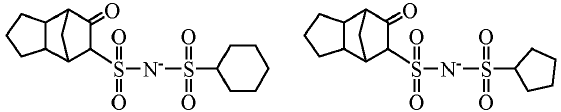

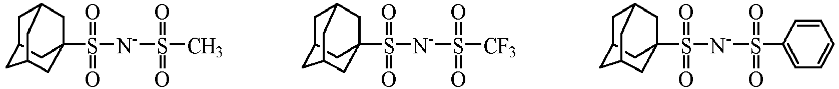

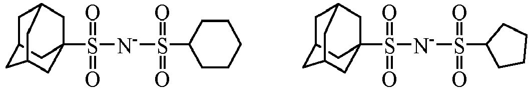

Exemplary structures for the onium salt include arbitrary combinations of anions with cations, both as exemplified above.

The onium salt having formula (A) may be synthesized by a combination of well-known organic chemistry methods, for example, according to the scheme shown below. 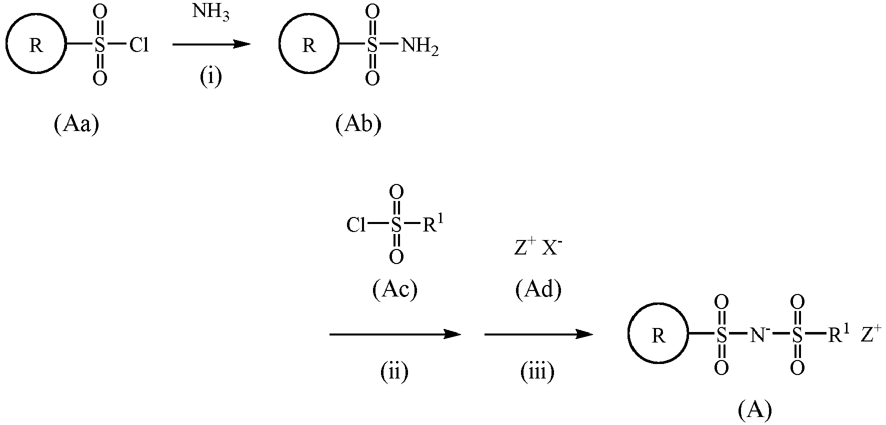 Herein ring R, R¹, and Z⁺ are as defined above, and X⁻ is a halide ion.

First, in step (i), a sulfonyl chloride compound (Aa) is reacted with ammonia to synthesize a sulfonamide compound (Ab). The reaction may be carried out, for example, by dissolving sulfonyl chloride compound (Aa) in a solvent, adding ammonia water thereto or blowing ammonia gas therein, and optionally heating or cooling the solution.

Examples of the solvent which can be used in the reaction of step (i) include water, ethers such as tetrahydrofuran (THF), diethyl ether, diisopropyl ether, di-n-butyl ether, and 1,4-dioxane, hydrocarbons such as n-hexane, n-heptane, benzene, toluene, and xylene, aprotic polar solvents such as acetonitrile, dimethyl sulfoxide (DMSO), N,N-dimethylformamide (DMF), chlorinated organic solvents such as methylene chloride, chloroform and carbon tetrachloride. Any suitable one may be chosen from these solvents, depending on reaction conditions, while the solvents may be used alone or in admixture.

The amount of ammonia used is preferably 1 to 30 moles, more preferably 1 to 5 moles per mole of sulfonyl chloride compound (Aa).

In step (i), the reaction temperature is preferably -20°C to 80°C, more preferably 0 to 40°C, and the reaction time is preferably 5 minutes to 24 hours, more preferably 1 to 20 hours.

Next, in step (ii), sulfonamide compound (Ab) is reacted with a sulfonyl chloride compound (Ac). Examples of the solvent which can be used in step (ii) include ethers such as THF, diethyl ether, diisopropyl ether, di-n-butyl ether, and 1,4-dioxane, hydrocarbons such as n-hexane, n-heptane, benzene, toluene, and xylene, aprotic polar solvents such as acetonitrile, DMSO, DMF, and chlorinated organic solvents such as methylene chloride, chloroform and carbon tetrachloride. Any suitable one may be chosen from these solvents, depending on reaction conditions, while the solvents may be used alone or in admixture.

For promoting the reaction, there may be co-present a base such as pyridine, triethylamine, 2,6-lutidine, or collidine. For promoting the reaction, it is also preferred to add 4-dimethylaminopyridine (DMAP).

The amount of sulfonyl chloride compound (Ac) used is preferably 1 to 30 moles, more preferably 1 to 5 moles per mole of sulfonamide compound (Ab).

In step (ii), the reaction temperature is preferably -20°C to 80°C, more preferably 0 to 40°C, and the reaction time is preferably 5 minutes to 24 hours, more preferably 1 to 20 hours.

Finally, in step (iii), ion exchange reaction is carried out between the reaction product of step (ii) and an onium salt (Ad) to synthesize the desired onium salt (A).

### Negative resist composition

The negative resist composition of the invention comprises (A) the onium salt defined above and (B) a base polymer.

The onium salt functions as a quencher in the negative resist composition. The quencher function mechanism is considered as follows. The acid generated by an acid generator in the resist composition in the exposed region should have a strong acidity enough to render the base polymer alkali insoluble. For example, α-fluorinated sulfonic acid, imide acid (or imidic acid) and methide acid are used in the ArF lithography. In a resist composition system where the acid generator and the onium compound co-exist, the acid generated by the acid generator undergoes ion exchange with the onium salt to convert to a sulfonium or iodonium salt again and instead, the anion of the onium salt is released as carboxylic acid. Differently stated, by ion exchange, the strong acid generated by the acid generator is neutralized with the onium salt. That is, the onium salt functions as a quencher. Another mechanism that the cation of the sulfonimide salt is photo-decomposed to generate sulfonimide acid is contemplated. However, the sulfonimide acid is a weak acid, which contributes little to the reaction to render the base polymer alkali insoluble.

The onium salt type quencher tends to form a resist pattern with a reduced LER as compared with the conventional quenchers in the form of amine compounds. This is presumably because salt exchange between strong acid and the onium salt type quencher is infinitely repeated. The site where strong acid is generated at the end of exposure shifts from the site where the onium salt of strong acid generation type is initially present. It is believed that since the cycle of photo-acid generation and salt exchange is repeated many times, the acid generation point is averaged, and this smoothing effect acts to reduce the LER of a resist pattern after development.

It is also believed that the quencher is, of course, present in the unexposed region as well. The quencher traps the acid that has diffused thereto from the exposed region via ion exchange reaction. This ensures to quench the acid that has diffused to the unexposed region. Then the contrast between exposed and unexposed regions is enhanced, leading to substantial improvements in resolution and LER.

As the compound that exerts a quencher effect via the same mechanism, Patent Documents 5 and 8 report the use of carboxylic acid onium salts, alkanesulfonic acid onium salts, and arenesulfonic acid onium salts as the quencher. On use of an alkanesulfonic acid onium salt or arenesulfonic acid onium salt, the generated acid has such an acid strength that part thereof in the overexposed region may induce alkali-insolubilizing reaction of the base polymer rather than the quencher, leading to an increase of acid diffusion, which invite degradation of resist performance factors like resolution and LER.

Also in the case of alkanecarboxylic acid onium salt, the carboxylic acid generated therefrom has a weak acidity and is substantially ineffective for rendering the base polymer alkali insoluble. However, the strong acid (from the acid generator) that has diffused to the unexposed region is not completely trapped, with the values of resolution and LER being unsatisfactory. In contrast, the quencher used in the inventive negative resist composition has a satisfactory quenching ability and more effectively traps the acid that has diffused to the unexposed region than the alkanecarboxylic acid onium salt.

Patent Document 9 describes a bis(p-toluenesulfonyl)imide salt. Since this compound is inferior in bulkiness to the inventive onium salt, its acid diffusion controlling ability is unsatisfactory as compared with the onium salt used in the inventive negative resist composition.

Since the onium salt is a salt compound, it is effective for preventing chemical flare by acid upon EB writing and does not volatilize by local heat during high current EB writing. Accordingly, it exerts the quencher function in the written region. Also, since the onium salt is retained in the non-written region without volatilization, it prevents unnecessary negative toning and is effective for reducing defects.

An appropriate amount of the onium salt (A) is 0.1 to 50 parts, more preferably 0.5 to 30 parts by weight per 100 parts by weight of the base polymer (B). As long as its amount is in the range, the onium salt fully functions as a quencher, eliminating any performance problems such as sensitivity drop, solubility shortage, and foreign particles. The onium salt (A) may be used alone or in admixture of two or more.

### (B) Base polymer

The negative resist composition also comprises (B) a base polymer containing a polymer comprising recurring units having the formula (B1). It is noted that the recurring unit having formula (B1) is simply referred to as recurring unit (B1), and the polymer is referred to as polymer (B). The recurring units (B1) are effective for imparting etching resistance, adhesion to substrates, and solubility in alkaline developer.

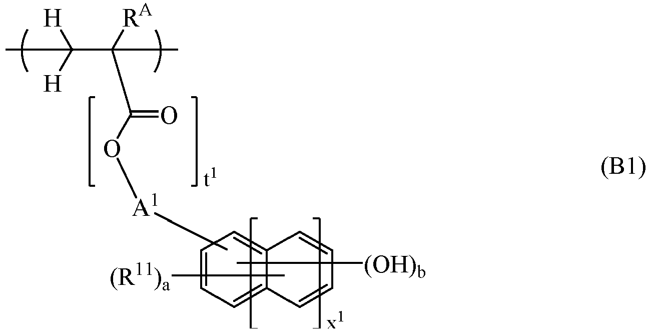

In formula (B1), R^{A} is hydrogen, fluorine, methyl or trifluoromethyl, with hydrogen or methyl being preferred. R¹¹ is each independently halogen, an optionally halogenated C₂-C₈ acyloxy group, optionally halogenated C₁-C₆ alkyl group, or optionally halogenated C₁-C₆ alkoxy group. A¹ is a single bond or a C₁-C₁₀ alkanediyl group in which an ether bond may intervene in a carbon-carbon bond, t¹ is 0 or 1, x¹ is an integer of 0 to 2, a is an integer satisfying 0 ≤ a ≤ 5+2x¹-b, and b is an integer of 1 to 5.

Examples of the alkanediyl group represented by A¹ include methylene, ethylene, propylene, butylene, pentylene, hexylene, and structural isomers of a carbon skeleton having branched or cyclic structure. For the alkanediyl group containing an ether bond, in case t¹=1 in formula (B1), the ether bond may be incorporated at any position excluding the position between the α- and β-carbons relative to the ester oxygen. In case t¹=0, the atom in A¹ that bonds with the main chain becomes an ethereal oxygen atom, and a second ether bond may be incorporated at any position excluding the position between the α- and β-carbons relative to that ether bond. Alkanediyl groups having no more than 10 carbon atoms are desirable because of a sufficient solubility in alkaline developer.

Preferred examples of the hydrocarbon portion in the acyloxy, alkyl and alkoxy groups represented by R¹¹ include methyl, ethyl, propyl, isopropyl, butyl, pentyl, hexyl and structural isomers of a carbon skeleton having branched or cyclic structure. As long as the carbon count is within the upper limit, good solubility in alkaline developer is available.

In formula (B1), x¹ is an integer of 0 to 2. The corresponding structure represents a benzene skeleton when x¹=0, a naphthalene skeleton when x¹=1, and an anthracene skeleton when x¹=2. The subscript a is an integer in the range: 0 ≤ a ≤ 5+2x¹-b. In case x¹=0, preferably a is an integer of 0 to 3, and b is an integer of 1 to 3. In case x¹=1 or 2, preferably a is an integer of 0 to 4, and b is an integer of 1 to 3.

Preferred examples of the recurring units (B1) wherein t¹=0 and A¹ is a single bond (meaning that the aromatic ring is directly bonded to the main chain of the polymer), that is, recurring units free of a linker (-CO-O-A¹-) include units derived from 3-hydroxystyrene, 4-hydroxystyrene, 5-hydroxy-2-vinylnaphthalene, and 6-hydroxy-2-vinylnaphthalene. More preferred are recurring units represented by the following formula (B1-1). 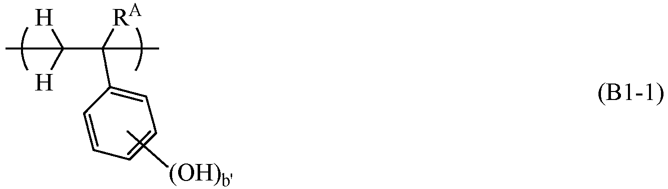 Herein R^{A} is as defined above and b' is an integer of 1 to 3.

Preferred examples of the recurring units (B1) wherein t¹=1, that is, having a linker (-CO-O-A¹-) are shown below, but not limited thereto. Herein R^{A} is as defined above.

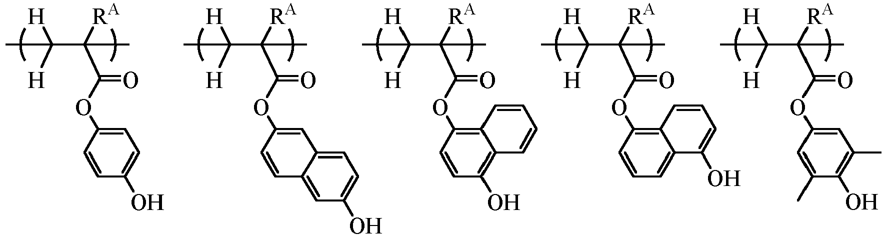

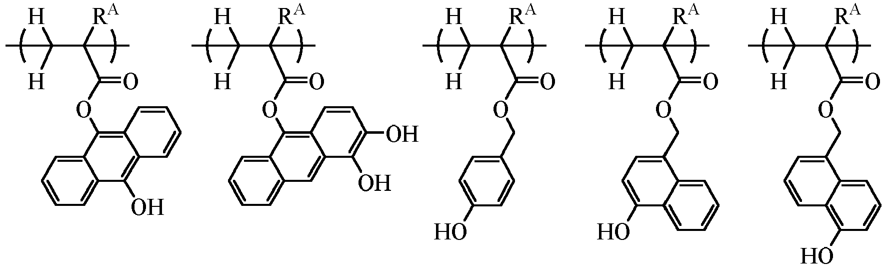

For the purpose of improving etch resistance, preferably the polymer (B) further comprises recurring units of at least one type selected from recurring units having the formula (B2), recurring units having the formula (B3) and recurring units having the formula (B4). Notably these units are simply referred to as recurring units (B2), (B3) and (B4).

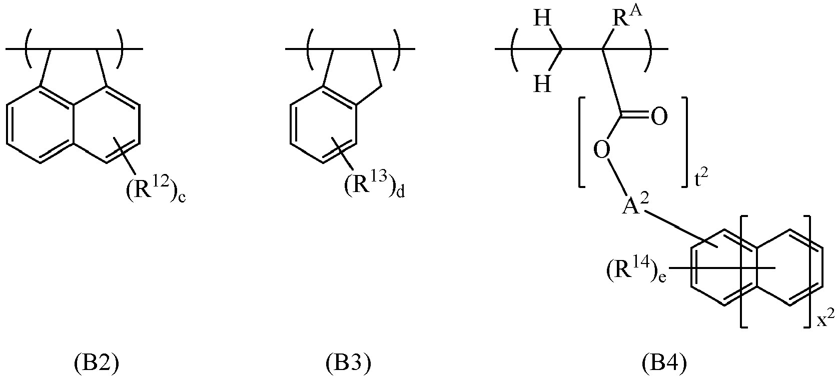

Herein R^{A} is as defined above. R¹² and R¹³ are each independently a hydroxyl group, halogen atom, acetoxy group, optionally halogenated C₁-C₈ alkyl group, optionally halogenated C₁-C₈ primary alkoxy group, C₂-C₈ secondary alkoxy group, optionally halogenated C₂-C₈ acyloxy group, or optionally halogenated C₂-C₈ alkylcarbonyloxy group. R¹⁴ is an acetyl group, acetoxy group, C₁-C₂₀ alkyl group, C₁-C₂₀ primary alkoxy group, C₂-C₂₀ secondary alkoxy group, C₂-C₂₀ acyloxy group, C₂-C₂₀ alkoxyalkyl group, C₂-C₂₀ alkylthioalkyl group, halogen atom, nitro group, or cyano group. A² is a single bond or C₁-C₁₀ alkanediyl group in which an ether bond may intervene in a carbon-carbon bond, c and d are each independently an integer of 0 to 4, e is an integer of 0 to 5, x² is an integer of 0 to 2, and t² is 0 or 1.

Preferred examples of the alkanediyl group represented by A² include methylene, ethylene, propylene, butylene, pentylene, hexylene and structural isomers of a carbon skeleton having branched or cyclic structure. For the alkanediyl group containing an ether bond, in case t²=1 in formula (B4), the ether bond may be incorporated at any position excluding the position between the α- and β-carbons relative to the ester oxygen. In case t²=0 in formula (B4), the atom in A² that bonds with the main chain becomes an ethereal oxygen atom, and a second ether bond may be incorporated at any position excluding the position between the α- and β-carbons relative to that ether bond. Alkanediyl groups having no more than 10 carbon atoms are desirable because of a high solubility in alkaline developer.

Preferred examples of the hydrocarbon portion in the alkyl, alkoxy, acyloxy and alkylcarbonyloxy groups represented by R¹² and R¹³ include methyl, ethyl, propyl, isopropyl, butyl, pentyl, hexyl and structural isomers of a carbon skeleton having branched or cyclic structure. As long as the carbon count is within the upper limit, good solubility in alkaline developer is available.

Preferred examples of the group R¹⁴ include chlorine, bromine, iodine, methyl, ethyl, propyl, butyl, pentyl, hexyl and structural isomers thereof, cyclopentyl, cyclohexyl, methoxy, ethoxy, propoxy, butoxy, pentyloxy, hexyloxy and structural isomers of its hydrocarbon moiety, cyclopentyloxy, and cyclohexyloxy. Inter alia, methoxy and ethoxy are useful. An acyloxy group may be readily introduced into a polymer even after polymerization, by a chemical modification method and is advantageously utilized for fine adjustment of the solubility of the base polymer in alkaline developer. Preferred acyloxy groups include methylcarbonyloxy, ethylcarbonyloxy, propylcarbonyloxy, butylcarbonyloxy, pentylcarbonyloxy, hexylcarbonyloxy and structural isomers thereof, cyclopentylcarbonyloxy, cyclohexylcarbonyloxy, and benzoyloxy. As long as the carbon count is equal to or less than 20, an appropriate effect of controlling or adjusting (typically reducing) the solubility of the polymer in alkaline developer is obtainable, and the generation of scum or development defects may be suppressed. Of the foregoing preferred substituent groups, such substituent groups as chlorine, bromine, iodine, methyl, ethyl and methoxy are useful because corresponding monomers may be readily prepared.

In formula (B4), x² is an integer of 0 to 2. The corresponding structure represents a benzene skeleton when x²=0, a naphthalene skeleton when x²=1, and an anthracene skeleton when x²=2. In case x²=0, preferably e is an integer of 0 to 3; in case x²=1 or 2, preferably e is an integer of 0 to 4.

Preferred examples of the recurring units (B4) wherein t² is 0 and A² is a single bond (meaning that the aromatic ring is directly bonded to the main chain of the polymer), that is, recurring units free of the linker (-CO-O-A²-) include units derived from styrene, 4-chlorostyrene, 4-bromostyrene, 4-methylstyrene, 4-methoxystyrene, 4-acetoxystyrene, 2-hydroxypropylstyrene, 2-vinylnaphthalene, and 3-vinylnaphthalene.

Preferred examples of the recurring units (B4) wherein t² is 1, that is, having the linker (-CO-O-A²-) are shown below, but not limited thereto. R^{A} is as defined above.

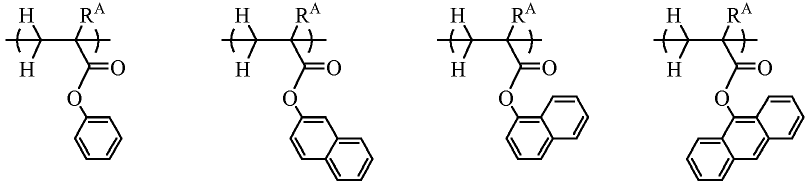

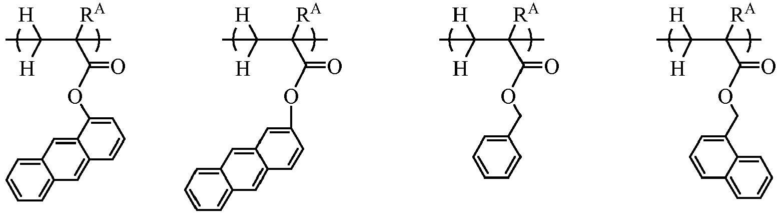

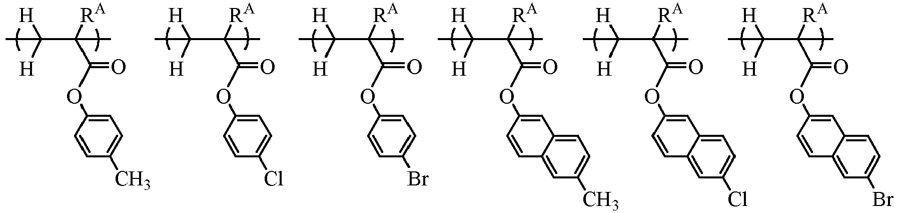

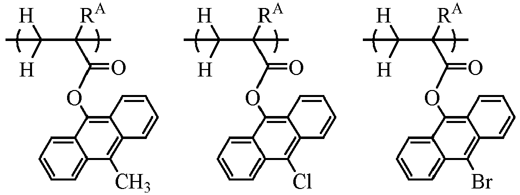

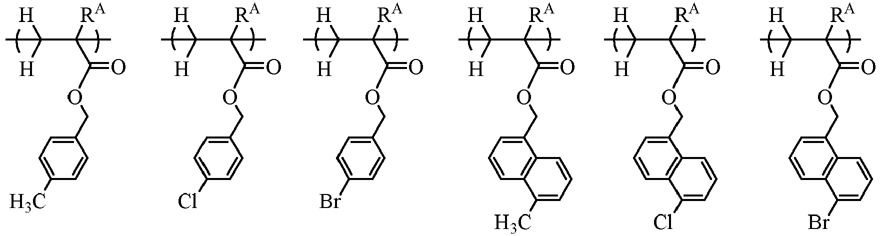

When recurring units of at least one type selected from recurring units (B2) to (B4) are incorporated, better performance is obtained because not only the aromatic ring possesses etching resistance, but the cyclic structure incorporated into the main chain also exerts the effect of improving resistance to EB irradiation during etching and pattern inspection steps.

The recurring units (B2) to (B4) may be of one type or a combination of plural types.

The polymer (B) may further comprise recurring units having the formula (B5). Notably the recurring units having formula (B5) are simply referred to as recurring units (B5), and of the polymers (B), a polymer further comprising recurring units (B5) is referred to as polymer (B').

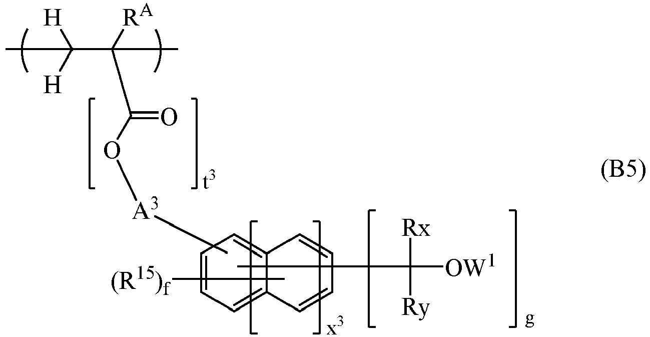

Herein R^{A} is as defined above. R¹⁵ is each independently halogen, an optionally halogenated C₂-C₈ acyloxy group, optionally halogenated C₁-C₆ alkyl group, or optionally halogenated C₁-C₆ alkoxy group. A³ is a single bond or C₁-C₁₀ alkanediyl group in which an ether bond may intervene in a carbon-carbon bond. W¹ is hydrogen, a C₁-C₁₀ monovalent aliphatic hydrocarbon group in which an ether bond, carbonyl or carbonyloxy moiety may intervene in a carbon-carbon bond, or an optionally substituted monovalent aromatic group. Rx and Ry are each independently hydrogen, a C₁-C₁₅ alkyl group which may be substituted with a hydroxy or alkoxy moiety, or an optionally substituted monovalent aromatic group, excluding that Rx and Ry are hydrogen at the same time. Rx and Ry may bond together to form a ring with the carbon atom to which they are attached. The subscript x³ is an integer of 0 to 2, t³ is 0 or 1, f is an integer in the range: 0 ≤ f ≤ 5+2x³-g, and g is an integer of 1 to 3.

Upon exposure to high-energy radiation, the unit (B5) functions such that the acid-eliminatable group undergoes elimination reaction under the action of an acid which is generated by the acid generator. That is, the unit (B5) induces alkali insolubilization and crosslinking reaction between polymer molecules.

Examples of the monovalent aliphatic hydrocarbon and monovalent aromatic groups represented by W¹ include methyl, ethyl, propyl, isopropyl, cyclopentyl, cyclohexyl, adamantyl, methylcarbonyl and phenyl.

Preferred structures of Rx and Ry include methyl, ethyl, propyl, butyl and structural isomers thereof, and substituted forms of the foregoing in which some hydrogen is substituted by hydroxy or alkoxy.

The subscript x³ is an integer of 0 to 2. The structure represents a benzene ring when x³=0, a naphthalene ring when x³=1, and an anthracene ring when x³=2.

Preferred examples of the alkanediyl group represented by A³ include methylene, ethylene, propylene, butylene, pentylene, hexylene and structural isomers of a carbon skeleton having branched or cyclic structure. For the alkanediyl group containing an ether bond, in case t³=1 in formula (B5), the ether bond may be incorporated at any position excluding the position between the α- and β-carbons relative to the ester oxygen. In case t³=0, the atom in A³ that bonds with the main chain becomes an ethereal oxygen atom, and a second ether bond may be incorporated at any position excluding the position between the α- and β-carbons relative to that ether bond. Alkanediyl groups having no more than 10 carbon atoms are desirable because of a high solubility in alkaline developer.

Preferred examples of the hydrocarbon portion in the acyloxy, alkyl and alkoxy groups represented by R¹⁵ include methyl, ethyl, propyl, isopropyl, butyl, pentyl, hexyl and structural isomers of a carbon skeleton having branched or cyclic structure. As long as the carbon count is within the upper limit, good solubility in alkaline developer is available.

Of the recurring units (B5), recurring units having formula (B5-1) or (B5-2) are preferred. 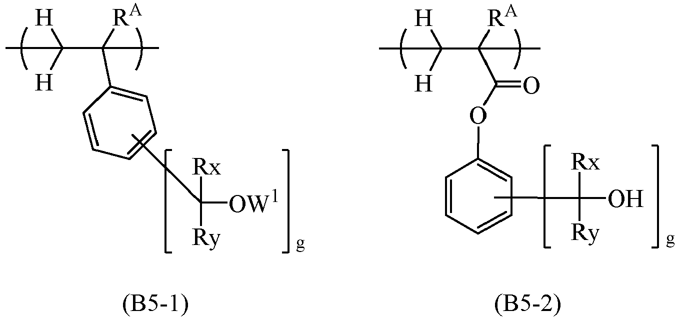 Herein R^{A}, Rx, Ry, W¹ and g are as defined above.

Preferred examples of the recurring unit (B5) are given below, but not limited thereto. Herein R^{A} is as defined above.

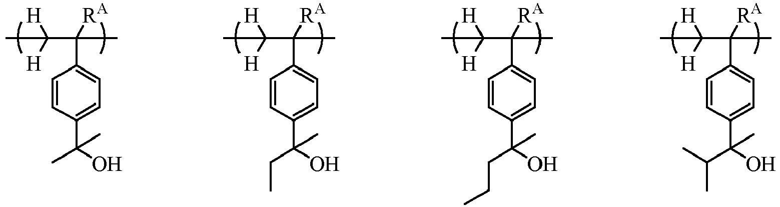

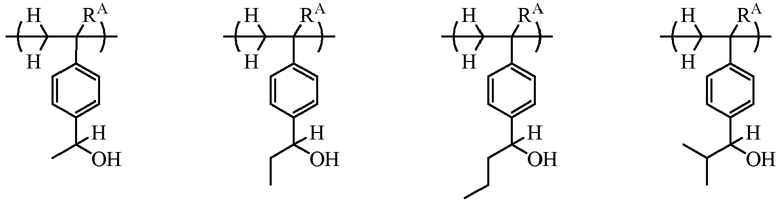

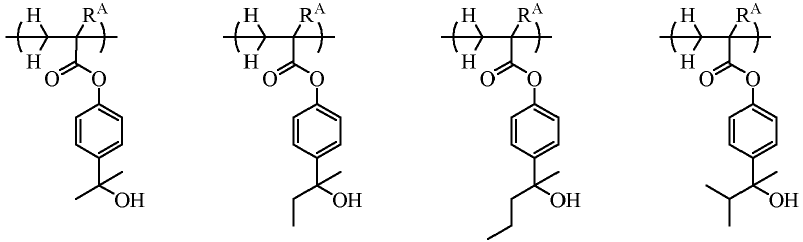

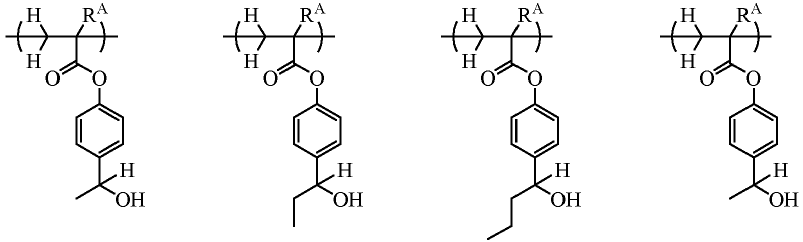

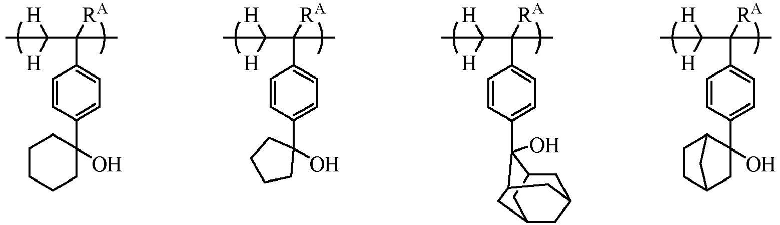

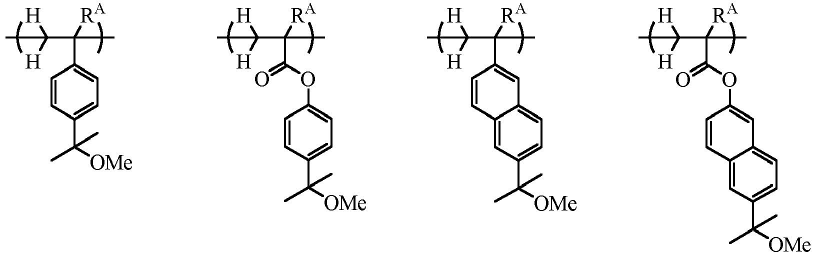

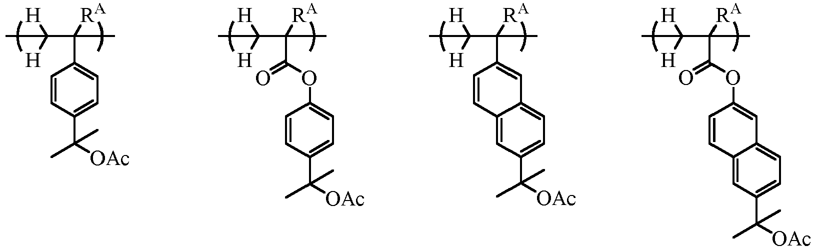

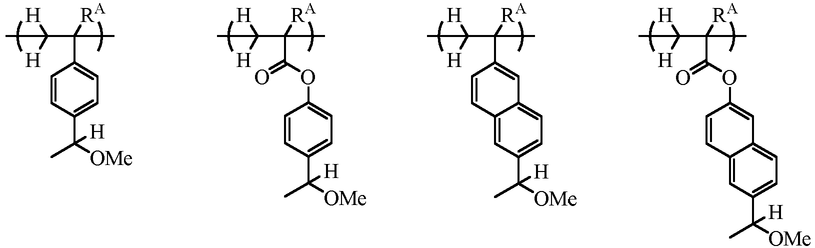

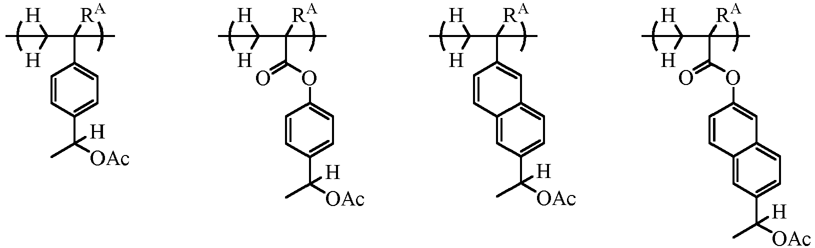

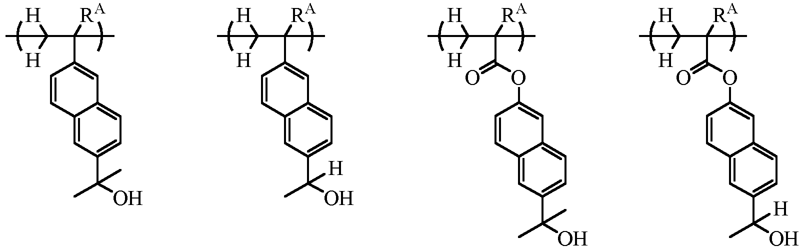

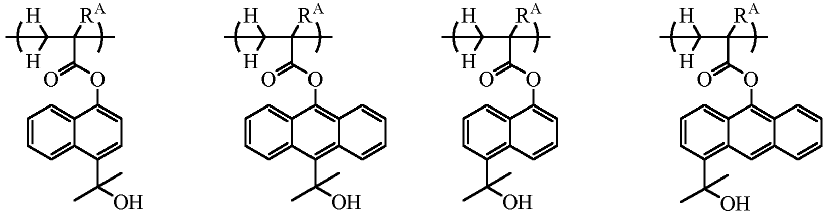

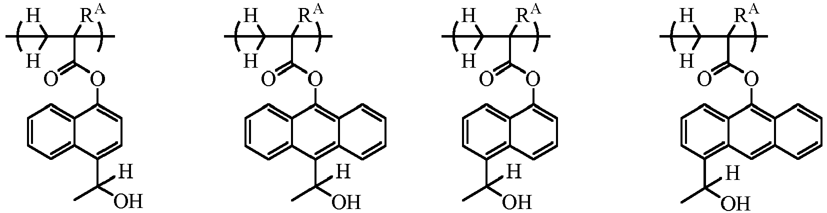

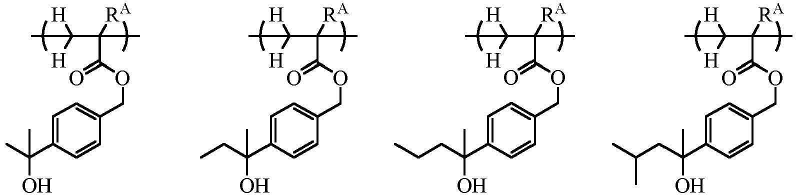

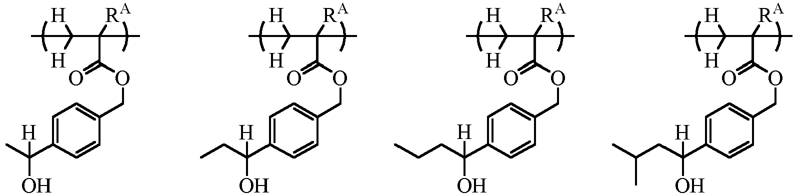

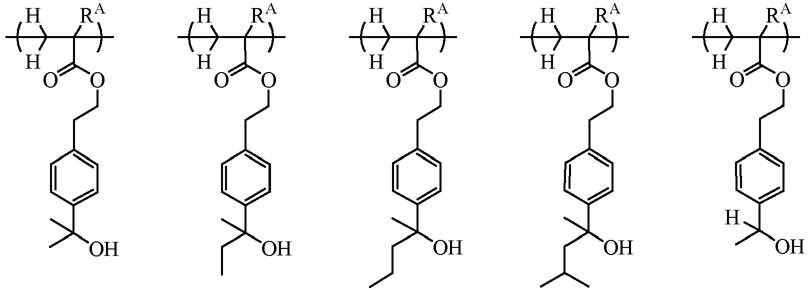

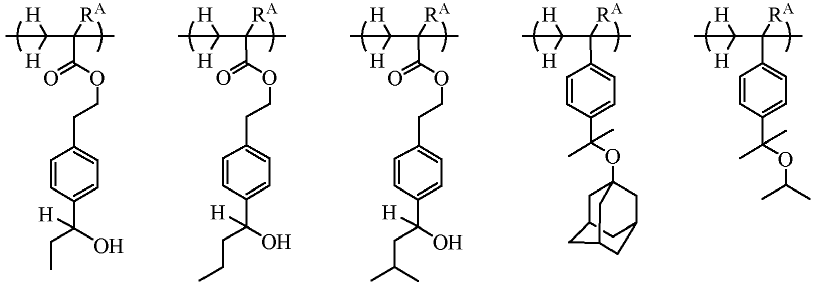

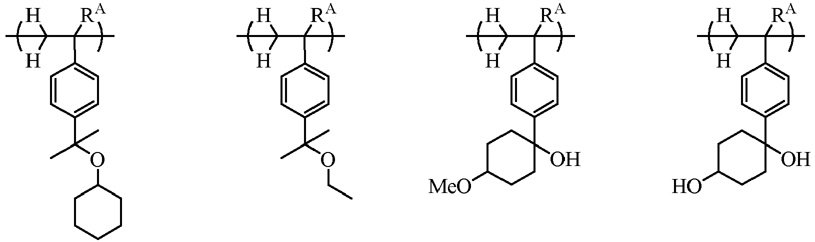

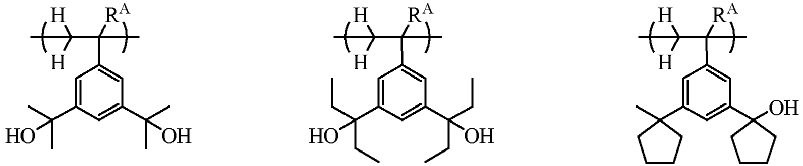

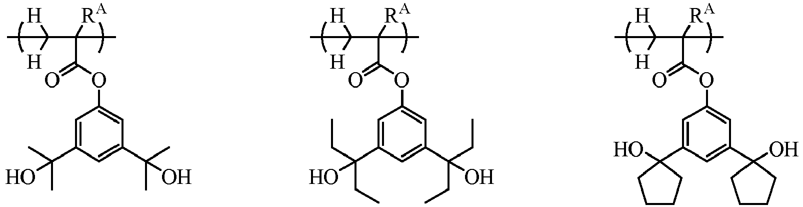

The polymer (B') may further comprise recurring units of at least one type selected from recurring units having formulae (B6) to (B8). Notably these recurring units are simply referred to as recurring units (B6) to (B8).

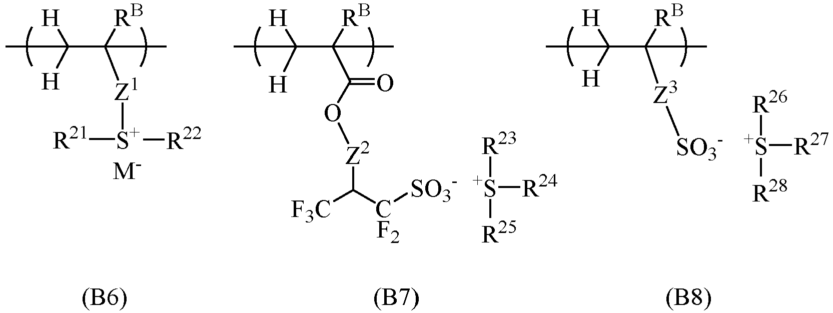

In formulae (B6) to (B8), R^{B} is each independently hydrogen or methyl. Z¹ is a single bond, phenylene group, -O-Z¹¹-, -C(=O)-O-Z¹¹-, or -C(=O)-NH-Z¹¹-, wherein Z¹¹ is a C₁-C₆ alkanediyl, C₂-C₆ alkenediyl or phenylene group, which may contain a carbonyl moiety, ester bond, ether bond or hydroxy moiety. Z² is a single bond or -Z²¹-C(=O)-O-, wherein Z²¹ is a C₁-C₂₀ divalent hydrocarbon group which may contain a heteroatom. Z³ is a single bond, methylene, ethylene, phenylene, fluorinated phenylene, -O-Z³¹-, -C(=O)-O-Z³¹-, or -C(=O)-NH-Z³¹-, wherein Z³¹ is a C₁-C₆ alkanediyl, C₂-C₆ alkenediyl or phenylene group, which may contain a carbonyl moiety, ester bond, ether bond or hydroxy moiety.

In formulae (B6) to (B8), R²¹ to R²⁸ are each independently a C₁-C₂₀ monovalent hydrocarbon group which may contain a heteroatom or in which some hydrogen may be substituted by a heteroatom such as oxygen, sulfur, nitrogen or halogen, or in which a heteroatom such as oxygen, sulfur or nitrogen may intervene in a carbon-carbon bond, so that the group may contain a hydroxyl, cyano, carbonyl, ether bond, ester bond, sulfonic acid ester bond, carbonate bond, lactone ring, sultone ring, carboxylic anhydride, or haloalkyl moiety. R²¹ and R²² may bond together to form a ring with the sulfur atom to which they are attached, any two or more of R²³, R²⁴ and R²⁵ may bond together to form a ring with the sulfur atom to which they are attached, and any two or more of R²⁶, R²⁷ and R²⁸ may bond together to form a ring with the sulfur atom to which they are attached.

In formula (B6), M⁻ is a non-nucleophilic counter ion.

In formula (B7) wherein Z² is -Z²¹-C(=O)-O-, Z²¹ is a divalent hydrocarbon group which may contain a heteroatom. Illustrative, non-limiting examples of the hydrocarbon group Z²¹ are given below.

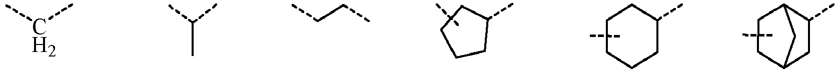

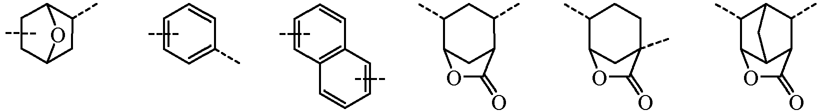

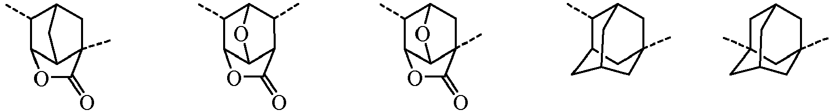

Examples of the sulfonium cation in formulae (B7) and (B8) wherein any two or more of R²³, R²⁴ and R²⁵ or any two or more of R²⁶, R²⁷ and R²⁸ bond together to form a ring with the sulfur atom to which they are attached, are shown below, but not limited thereto. 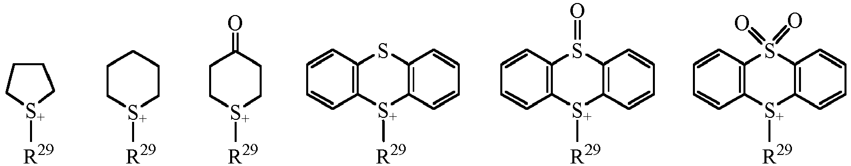 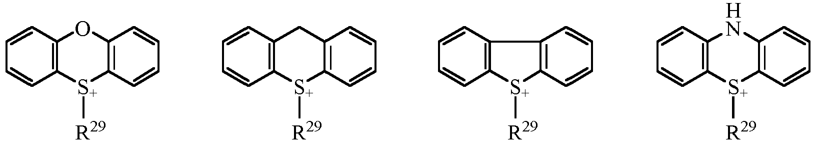 It is noted that R²⁹ is the same as defined and exemplified for R²¹ to R²⁸.

Specific examples of the sulfonium cation in formulae (B7) and (B8) are shown below, but not limited thereto.

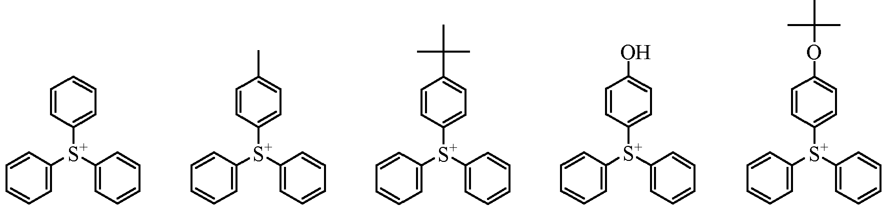

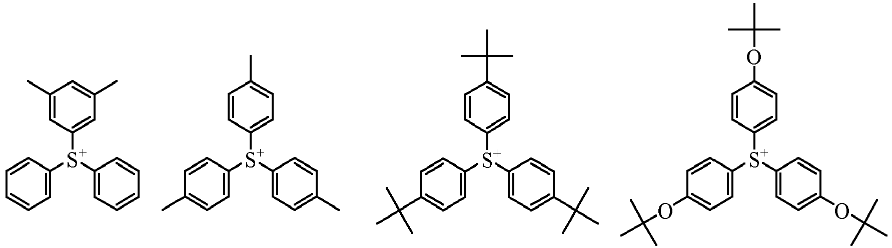

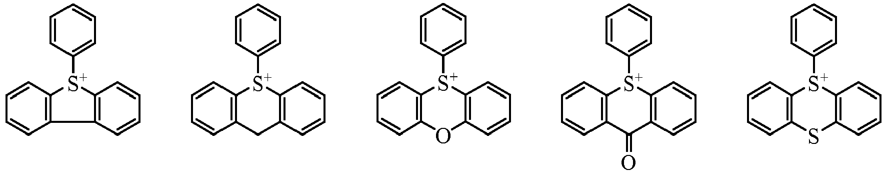

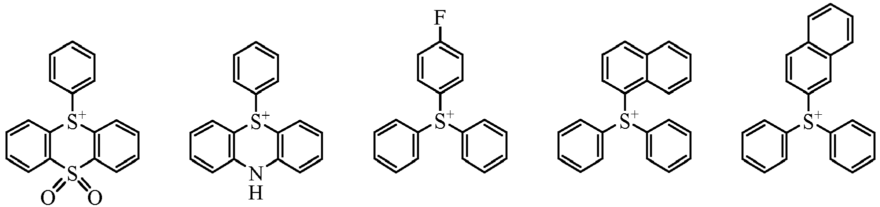

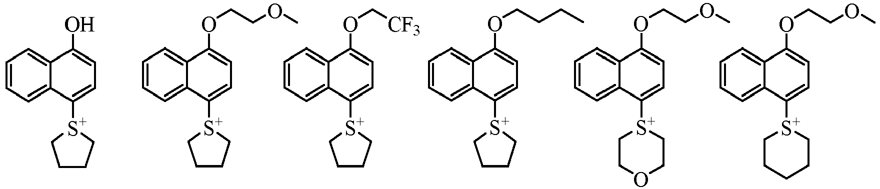

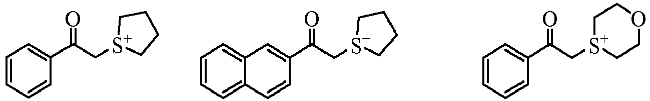

The recurring units (B6) to (B8) are units capable of generating an acid upon receipt of high-energy radiation. With the relevant units bound into a polymer, an appropriate control of acid diffusion becomes possible, and a pattern with minimal LER can be formed. Since the acid-generating unit is bound to a polymer, the phenomenon that acid volatilizes from the exposed region and re-deposits on the unexposed region during bake in vacuum is suppressed. This is effective for reducing LER and for suppressing unwanted negative tone reaction in the unexposed region for thereby reducing defects. The content of recurring units (B6) to (B8) is preferably 0.5 to 20 mol% based on the overall recurring units of polymer (B').

In the polymer, (meth)acrylate and other recurring units having an adhesive group such as lactone structure may be incorporated for fine adjustment of properties of a resist film, though they are optional.

In polymer (B), an appropriate content of recurring units (B1) is 50 to 95 mol%, more preferably 70 to 85 mol%; an appropriate content of recurring units (B2) to (B4) is 5 to 50 mol%, more preferably 15 to 30 mol%; an appropriate content of other recurring units is 0 to 20 mol%, more preferably 0 to 10 mol%.

Where the polymer (B') is free of recurring units (B6) to (B8), the polymer preferably contains 25 to 95 mol%, more preferably 40 to 85 mol% of recurring units (B1). An appropriate content of recurring units (B2) to (B4) is 0 to 30 mol%, more preferably 5 to 20 mol%. An appropriate content of recurring units (B5) is 5 to 45 mol%, more preferably 10 to 40 mol%. The other recurring units may be incorporated in a range of 0 to 20 mol%, preferably 0 to 10 mol%.

Where the polymer (B') contains recurring units (B6) to (B8), the polymer preferably contains 25 to 94.5 mol%, more preferably 50 to 85 mol% of recurring units (B1). An appropriate content of recurring units (B2) to (B4) is 0 to 20 mol%, more preferably 3 to 15 mol%. An appropriate content of recurring units (B5) is 5 to 35 mol%, more preferably 10 to 25 mol%. An appropriate content of recurring units (B6) to (B8) is 0.5 to 20 mol%, more preferably 2 to 10 mol%. The other recurring units may be incorporated in a range of 0 to 20 mol%, preferably 0 to 10 mol%.

Also preferably recurring units (B1) to (B5) account for at least 60 mol%, more preferably at least 70 mol% of the overall recurring units of the polymer. This range ensures that the polymer has necessary properties as the inventive negative resist composition.

As the polymer (B') containing recurring units (B6) to (B8), a polymer comprising recurring units having the formula (B1-1), recurring units having the formula (B5-1), and recurring units having the formula (B7) is preferred. 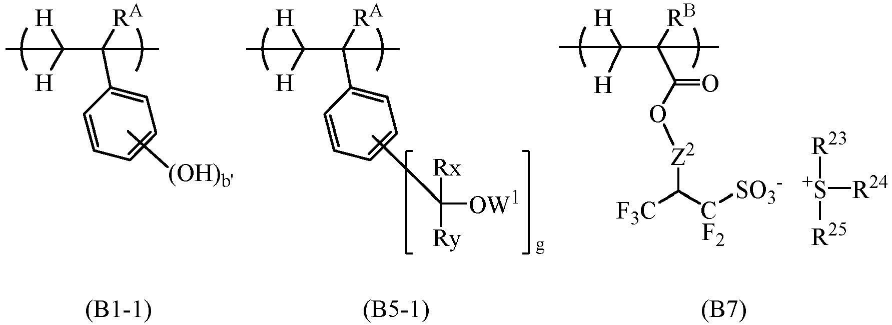 Herein R^{A}, R^{B}, Z², R²³, R²⁴, R²⁵, Rx, Ry, W¹, b', and g are as defined above.

Where the polymer (B') is used as the base polymer (B), it may be a mixture of a polymer free of recurring units (B6) to (B8) and a polymer comprising recurring units (B6) to (B8). In this embodiment, the polymer free of recurring units (B6) to (B8) is preferably used in an amount of 2 to 5,000 parts, more preferably 10 to 1,000 parts by weight per 100 parts by weight of the polymer comprising recurring units (B6) to (B8).

The polymer may be synthesized by combining suitable monomers optionally protected with a protective group, copolymerizing them in the standard way, and effecting deprotection reaction if necessary. The copolymerization reaction is preferably radical polymerization or anionic polymerization though not limited thereto. For the polymerization reaction, reference may be made to WO 2006/121096, JP-A 2004-115630, JP-A 2008-102383, and JP-A 2008-304590.

The polymer should preferably have a Mw of 2,000 to 50,000, and more preferably 3,000 to 20,000. A Mw of at least 2,000 eliminates the risk that pattern features are rounded at their top, inviting degradations of resolution and LER. A Mw of up to 50,000 eliminates the risk that LER is increased. A Mw of up to 20,000 is preferable particularly when a pattern with a line width of up to 100 nm is formed. As used herein, Mw is measured by GPC versus polystyrene standards.

The polymer preferably has a narrow molecular weight distribution or dispersity (Mw/Mn) of 1.0 to 2.0, more preferably 1.0 to 1.8. A polymer with such a narrow dispersity eliminates any foreign particles left on the pattern or profile degradation of the pattern after development.

### (C) Crosslinker

When only polymer (B) is used as the base polymer in the negative resist composition, a crosslinker is preferably added. When the base polymer contains polymer (B'), a crosslinker need not be added.

Suitable crosslinkers which can be used herein include melamine compounds, guanamine compounds, glycoluril compounds and urea compounds having substituted thereon at least one group selected from among methylol, alkoxymethyl and acyloxymethyl groups, epoxy compounds, isocyanate compounds, azide compounds, and compounds having a double bond such as an alkenyl ether group. These compounds may be used as an additive or introduced into a polymer side chain as a pendant. Hydroxy-containing compounds may also be used as the crosslinker.

Of the foregoing crosslinkers, examples of suitable epoxy compounds include tris(2,3-epoxypropyl) isocyanurate, trimethylolmethane triglycidyl ether, trimethylolpropane triglycidyl ether, and triethylolethane triglycidyl ether. Examples of the melamine compound include hexamethylol melamine, hexamethoxymethyl melamine, hexamethylol melamine compounds having 1 to 6 methylol groups methoxymethylated and mixtures thereof, hexamethoxyethyl melamine, hexaacyloxymethyl melamine, hexamethylol melamine compounds having 1 to 6 methylol groups acyloxymethylated and mixtures thereof. Examples of the guanamine compound include tetramethylol guanamine, tetramethoxymethyl guanamine, tetramethylol guanamine compounds having 1 to 4 methylol groups methoxymethylated and mixtures thereof, tetramethoxyethyl guanamine, tetraacyloxyguanamine, tetramethylol guanamine compounds having 1 to 4 methylol groups acyloxymethylated and mixtures thereof. Examples of the glycoluril compound include tetramethylol glycoluril, tetramethoxyglycoluril, tetramethoxymethyl glycoluril, tetramethylol glycoluril compounds having 1 to 4 methylol groups methoxymethylated and mixtures thereof, tetramethylol glycoluril compounds having 1 to 4 methylol groups acyloxymethylated and mixtures thereof. Examples of the urea compound include tetramethylol urea, tetramethoxymethyl urea, tetramethylol urea compounds having 1 to 4 methylol groups methoxymethylated and mixtures thereof, and tetramethoxyethyl urea.

Suitable isocyanate compounds include tolylene diisocyanate, diphenylmethane diisocyanate, hexamethylene diisocyanate and cyclohexane diisocyanate. Suitable azide compounds include 1,1'-biphenyl-4,4'-bisazide, 4,4'-methylidenebisazide, and 4,4'-oxybisazide. Examples of the alkenyl ether group-containing compound include ethylene glycol divinyl ether, triethylene glycol divinyl ether, 1,2-propanediol divinyl ether, 1,4-butanediol divinyl ether, tetramethylene glycol divinyl ether, neopentyl glycol divinyl ether, trimethylol propane trivinyl ether, hexanediol divinyl ether, 1,4-cyclohexanediol divinyl ether, pentaerythritol trivinyl ether, pentaerythritol tetravinyl ether, sorbitol tetravinyl ether, sorbitol pentavinyl ether, and trimethylol propane trivinyl ether.

An appropriate amount of the crosslinker used is 2 to 50 parts, and more preferably 5 to 30 parts by weight per 100 parts by weight of the base polymer. As long as the amount of the crosslinker is in the range, the risk of resolution being reduced by forming bridges between pattern features is mitigated. The crosslinkers may be used alone or in admixture.

### (D) Organic solvent

The negative resist composition may further comprise (D) an organic solvent. The organic solvent used herein is not particularly limited as long as the components are soluble therein. Examples of the organic solvent are described in JP-A 2008-111103, paragraphs [0144] to [0145] (USP 7,537,880). Specifically, exemplary solvents include ketones such as cyclohexanone and methyl-2-n-pentyl ketone; alcohols such as 3-methoxybutanol, 3-methyl-3-methoxybutanol, 1-methoxy-2-propanol, 1-ethoxy-2-propanol, and diacetone alcohol; ethers such as propylene glycol monomethyl ether, ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate, ethyl pyruvate, butyl acetate, methyl 3-methoxypropionate, ethyl 3-ethoxypropionate, t-butyl acetate, t-butyl propionate, and propylene glycol mono-t-butyl ether acetate; and lactones such as γ-butyrolactone, and mixtures thereof. Where an acid labile group of acetal form is used, a high-boiling alcohol solvent such as diethylene glycol, propylene glycol, glycerol, 1,4-butanediol or 1,3-butanediol may be added for accelerating deprotection reaction of acetal. Of the above organic solvents, it is recommended to use 1-ethoxy-2-propanol, PGMEA, propylene glycol monomethyl ether, cyclohexanone, ethyl lactate, γ-butyrolactone, and mixtures thereof.

An appropriate amount of the organic solvent (D) used is 200 to 10,000 parts, more preferably 400 to 5,000 parts by weight per 100 parts by weight of the base polymer (B).

### (E) Acid generator

The negative resist composition may further comprise (E) an acid generator in order that the composition function as a chemically amplified negative resist composition. The acid generator is typically a compound capable of generating acid in response to actinic light or radiation (known as photoacid generator). It may be any compound capable of generating an acid upon exposure to high-energy radiation. Suitable PAGs include sulfonium salts, iodonium salts, sulfonyldiazomethane, N-sulfonyloxyimide, and oxime-O-sulfonate acid generators. These PAGs may be used alone or in admixture of two or more.

Suitable PAGs include those described in JP-A 2008-111103, paragraphs [0122]-[0142]. Among others, arenesulfonate type PAGs are preferred because they generate acids having an appropriate strength to promote reaction of base polymer (B) with crosslinker (C). The PAG capable of generating an acid having a pKa value in the range of -3.0 to 1.5, more preferably -1.0 to 1.5 is preferred because the effect of improving LER by combining the generated acid with the onium salt (A) to induce exchange reaction is achievable.

The preferred PAGs are sulfonium salts having a sulfonate anion of the structure shown below. Notably the cation that pairs with the anion is as exemplified for the sulfonium cation in formulae (B7) and (B8).

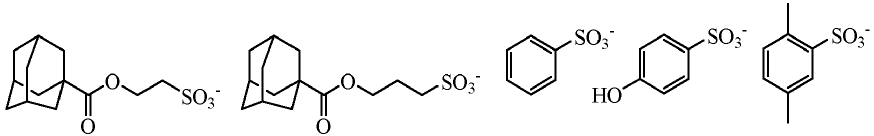

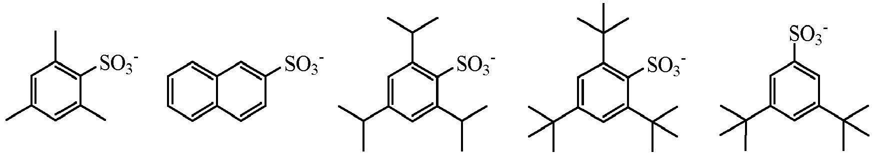

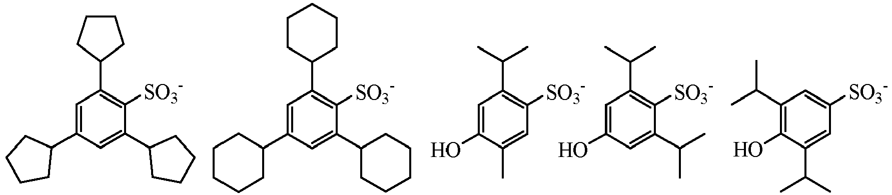

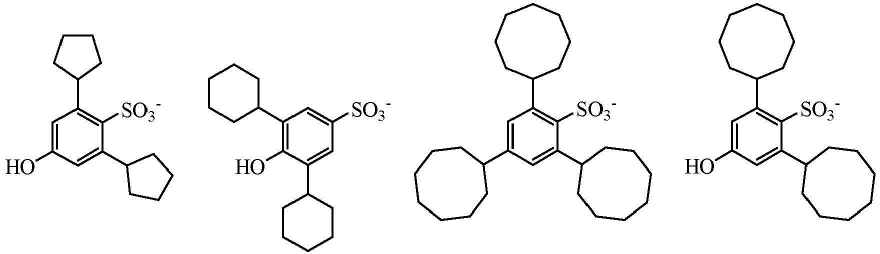

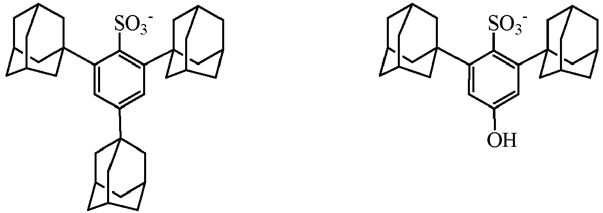

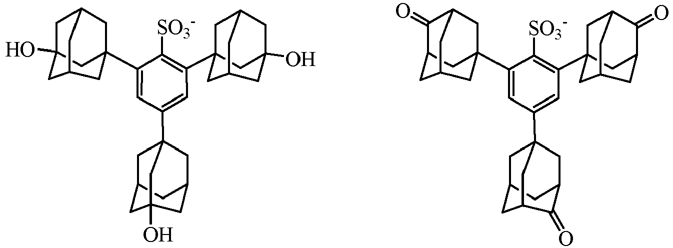

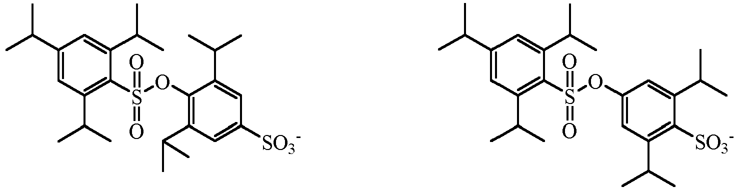

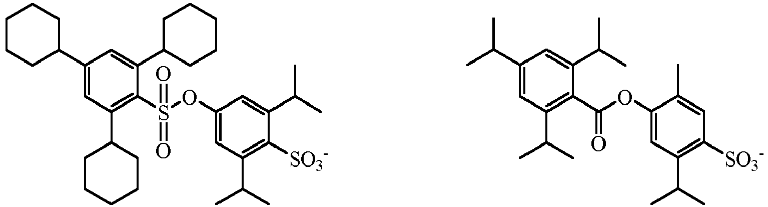

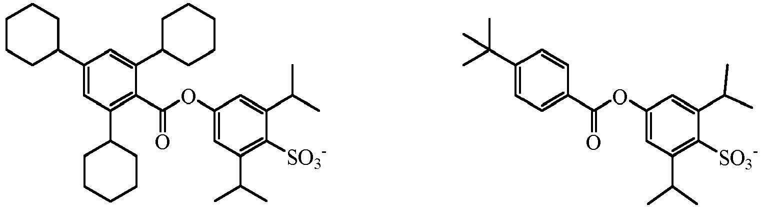

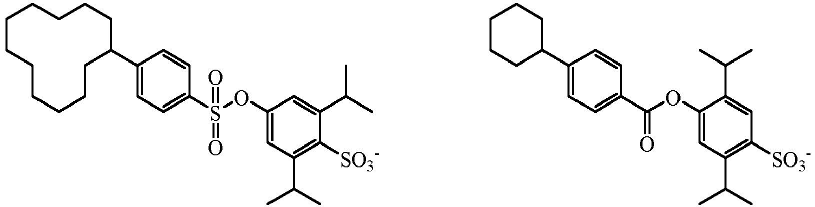

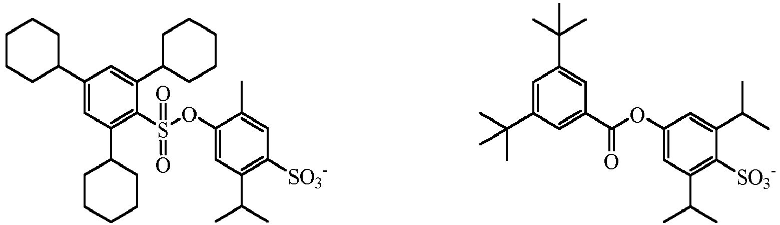

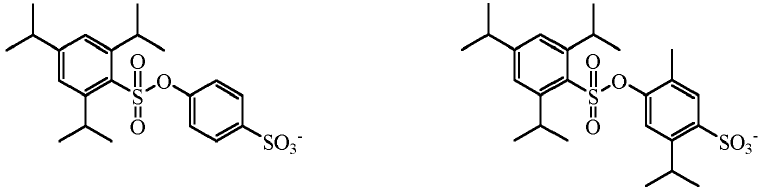

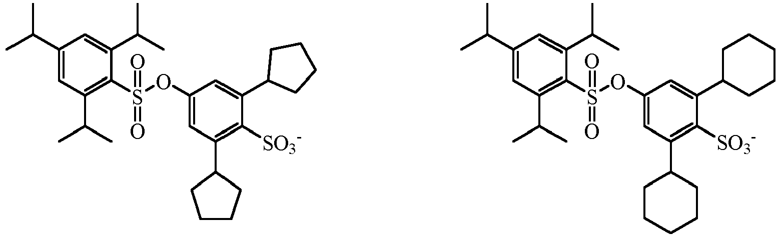

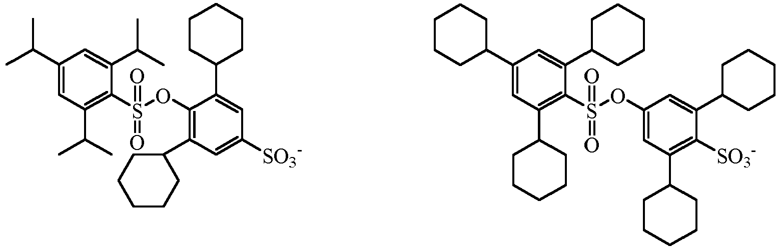

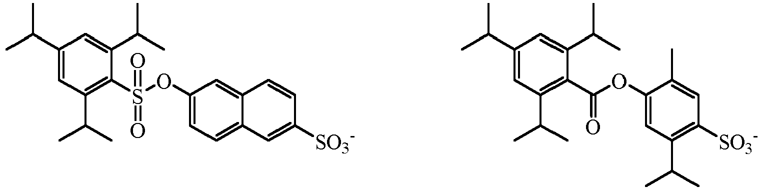

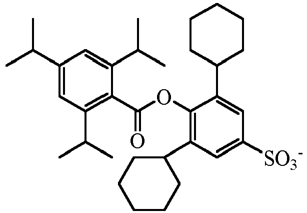

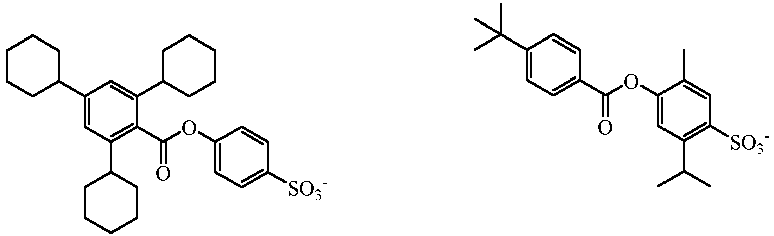

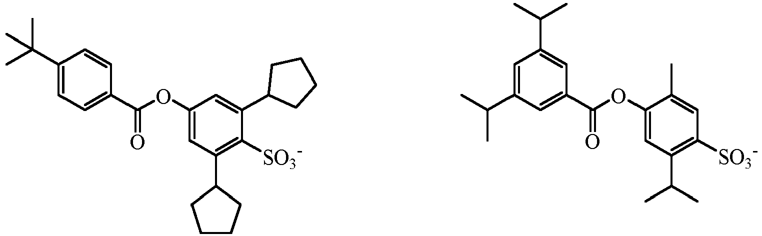

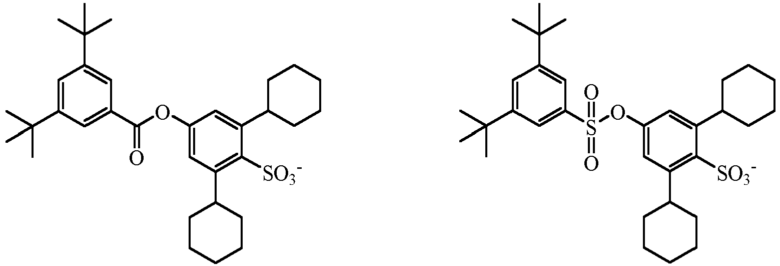

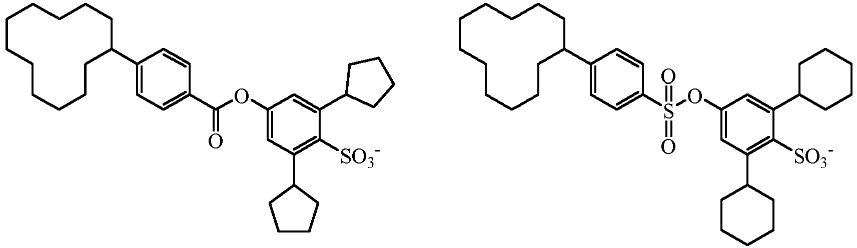

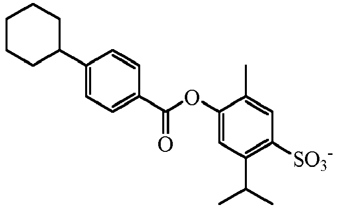

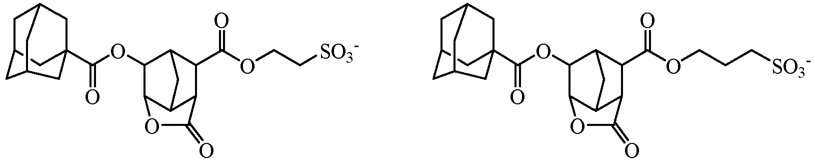

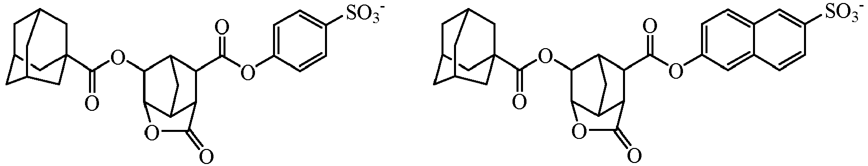

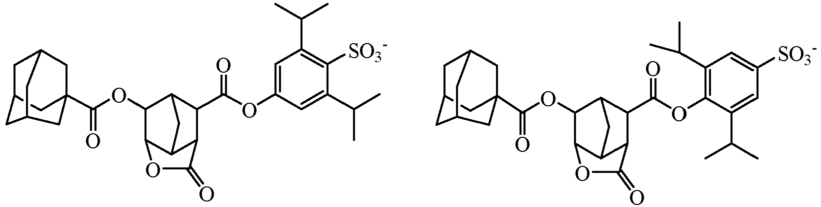

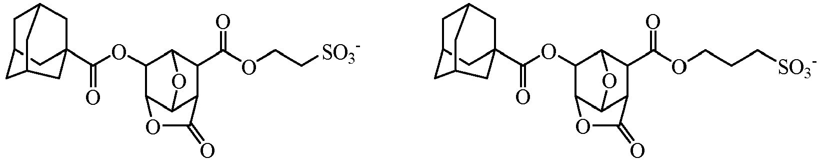

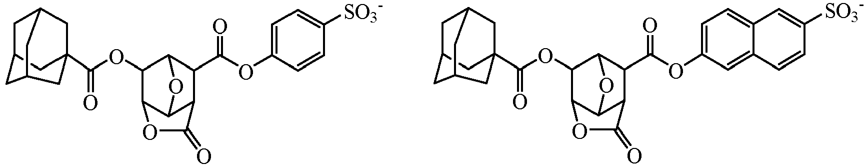

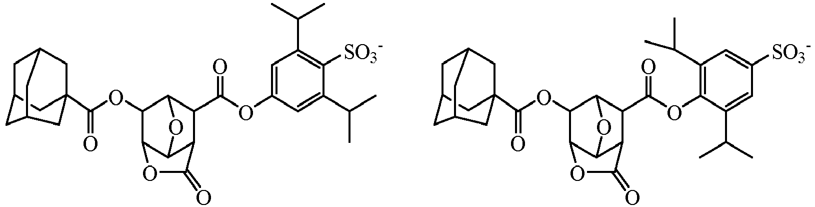

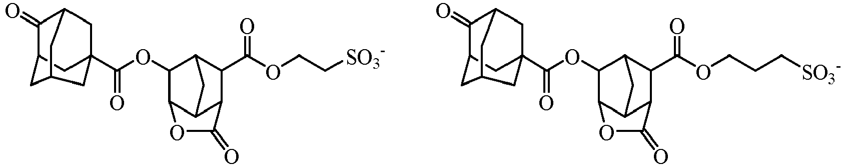

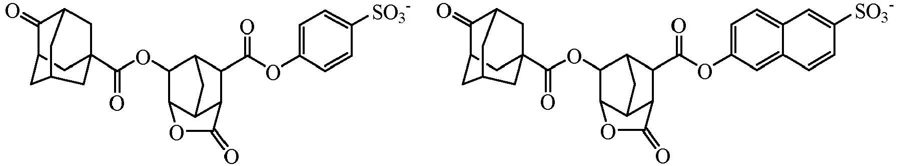

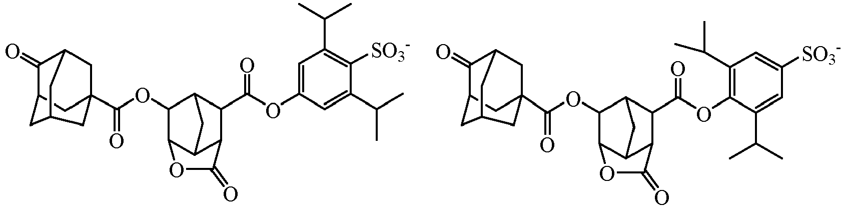

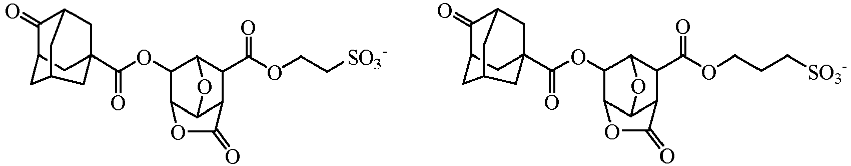

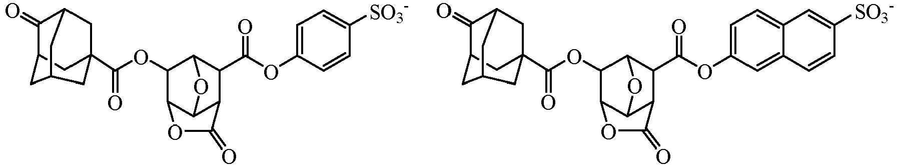

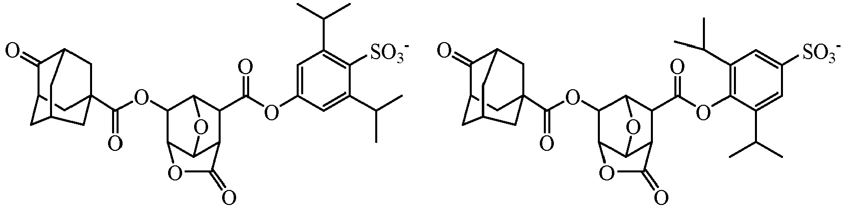

An appropriate amount of the acid generator (E) used is 2 to 20 parts, more preferably 5 to 15 parts by weight per 100 parts by weight of the base polymer (B). Where the base polymer contains recurring units (B6) to (B8), the PAG may be omitted.

### (F) Basic compound

In the resist composition, (F) a basic compound may be added as the quencher (other than component (A)) for the purpose of correcting a pattern profile or the like. The basic compound is effective for controlling acid diffusion. Even when the resist film is applied to a processable substrate having an outermost surface layer made of a chromium-containing material, the basic compound is effective for minimizing the influence of the acid generated in the resist film on the chromium-containing material.

Numerous basic compounds are known useful including primary, secondary, and tertiary aliphatic amines, mixed amines, aromatic amines, heterocyclic amines, nitrogen-containing compounds with carboxyl group, nitrogen-containing compounds with sulfonyl group, nitrogen-containing compounds with hydroxyl group, nitrogen-containing compounds with hydroxyphenyl group, alcoholic nitrogen-containing compounds, amide derivatives, imide derivatives, carbamate derivatives, and ammonium salts. Examples are described in JP 4575479, for example, and any such compounds are useful. Of the foregoing basic compounds, preferred are tris[2-(methoxymethoxy)ethyl]amine, tris[2-(methoxymethoxy)ethyl]amine-N-oxide, morpholine derivatives and imidazole derivatives.

An appropriate amount of the basic compound (F) added is 0 to 10 parts, and more preferably 0 to 5 parts by weight per 100 parts by weight of the base polymer (B). The basic compounds may be used alone or in admixture.

### (G) Surfactant

In the resist composition, any of surfactants commonly used for improving coating characteristics to the processable substrate may be added as an optional component. Numerous surfactants are known in the art including those described in JP-A 2004-115630, for example. A choice may be made with reference to such patent documents. An appropriate amount of the surfactant (G) used is 0 to 2 parts by weight per 100 parts by weight of the base polymer (B).

### Process

The invention also relates to a resist pattern forming process comprising the steps of applying the resist composition defined above onto a substrate to form a resist film thereon, exposing the resist film patternwise to high-energy radiation, and developing the resist film in an alkaline developer to form a resist pattern.

Pattern formation using the negative resist composition of the invention may be performed by well-known lithography processes. In general, the resist composition is first applied onto a processable substrate such as a substrate for IC fabrication (e.g., Si, SiO, SiO₂, SiN, SiON, TiN, WSi, BPSG, SOG, organic antireflective coating, etc.) or a substrate for mask circuit fabrication (e.g., Cr, CrO, CrON, MoSi₂, Si, SiO, SiO₂, etc.) by a suitable coating technique such as spin coating. The coating is prebaked on a hotplate at a temperature of 60 to 150°C for 1 to 20 minutes, preferably at 80 to 140°C for 1 to 10 minutes to form a resist film of 0.03 to 2 µm thick.

An anti-charging film comprising a conductive polymer may be formed on the resist film. The anti-charging film is effective for preventing a charging phenomenon or charge buildup in the resist film during EB writing for thereby achieving a significant improvement in writing position accuracy. The anti-charging film is typically formed of a conductive polymer such as polyaniline or polythiophene as described in JP-A 2016-200634.

Then the resist film is exposed patternwise to high-energy radiation such as UV, deep-UV, excimer laser (KrF, ArF), EUV, x-ray, γ-ray or synchrotron radiation or EB. On use of UV, deep-UV, EUV, excimer laser, x-ray, γ-ray or synchrotron radiation as the high-energy radiation, the resist film is exposed through a mask having a desired pattern, preferably in a dose of 1 to 200 mJ/cm², more preferably 10 to 100 mJ/cm². On use of EB, a pattern may be written directly or through a mask having the desired pattern, preferably in a dose of 1 to 300 µC/cm², more preferably 10 to 200 µC/cm². The resist composition of the invention is especially effective in the EUV or EB lithography.

The exposure may be performed by conventional lithography whereas the immersion lithography of holding a liquid between the mask and the resist film may be employed if desired. In the case of immersion lithography, a protective film which is insoluble in water may be used.

The resist film is then baked (PEB) on a hotplate at 60 to 150°C for 1 to 20 minutes, preferably at 80 to 140°C for 1 to 10 minutes. Thereafter the resist film is developed with a developer in the form of an aqueous base solution, for example, 0.1 to 5 wt%, preferably 2 to 3 wt% aqueous solution of tetramethylammonium hydroxide (TMAH) for 0.1 to 3 minutes, preferably 0.5 to 2 minutes by conventional techniques such as dip, puddle and spray techniques. In this way, a desired resist pattern is formed on the substrate.

From the resist composition, a pattern with a high resolution and minimal LER may be formed. The resist composition is effectively applicable to a substrate, specifically a substrate having a surface layer of material to which a resist film is less adherent and which is likely to invite pattern stripping or pattern collapse, and particularly a substrate having sputter deposited thereon metallic chromium or a chromium compound containing at least one light element selected from oxygen, nitrogen and carbon or a substrate having an outermost surface layer of SiOₓ. The invention is especially effective for pattern formation on a photomask blank as the substrate.

### EXAMPLES

Examples of the invention are given below by way of illustration and not by way of limitation. The abbreviation "pbw" is parts by weight. THF stands for tetrahydrofuran, and DMAP for dimethylaminopyridine. The copolymer composition is expressed by a molar ratio. Mw is measured versus polystyrene standards by GPC using THF solvent.

### [1] Synthesis of onium salt

Examples indicated by an asterisks (*) are intermediate Examples not falling within the claimed scope.

### Example 1-1*

### Synthesis of Salt-1

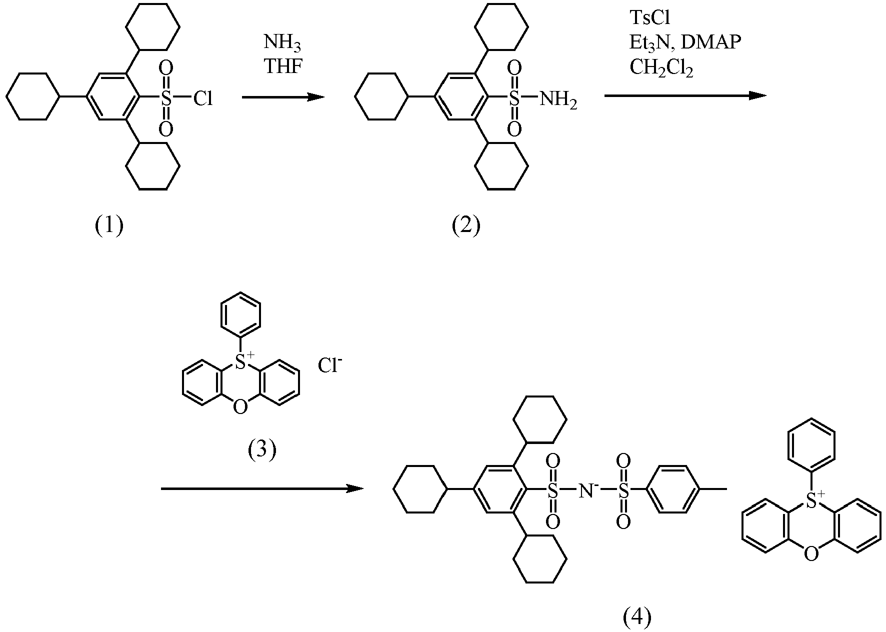

In 1,000 g of THF was dissolved 100 g of 2,4,6-tricyclohexylbenzenesulfonyl chloride (1). The solution was combined with 28.7 g of 28 wt% NH₃ aqueous solution and stirred at room temperature for 12 hours. Then 3,000 g of methylene chloride was added to the solution. The organic layer was taken out, washed with water, and concentrated under reduced pressure, obtaining 90.1 g of the desired compound, 2,4,6-tricyclohexylbenzenesulfonamide (2) as white solid (yield 95%).

In 200 g of methylene chloride (CH₂Cl₂) was dissolved 20 g of 2,4,6-tricyclohexylbenzenesulfonamide (2). Then 11.3 g of p-toluenesulfonyl chloride (TsCl) and 1.2 g of DMAP were added. Under ice cooling, 7.5 g of triethylamine (Et₃N) was added dropwise to the solution, which was stirred at room temperature for 6 hours. Thereafter, 180 g of 5 wt% HCl aqueous solution and 200 g of methylene chloride were added to the solution. The organic layer was taken out, washed with water, and combined with 517 g of 6 wt% 10-phenylphenoxathiinium chloride aqueous solution. The organic layer was taken out again, washed with water, and concentrated under reduced pressure. Toluene was added to the concentrate, followed by concentration again. The solid precipitate was washed with diisopropyl ether. The solid was dried in vacuum, obtaining 27.3 g of the target compound, 10-phenylphenoxathiinium 2,4,6-tricyclohexyl-N-(p-toluenesulfonyl)-benzenesulfonamidate (4) as white solid (yield 66%). This is designated Salt-1.

### Example 1-2*

### Synthesis of Salt-2

The same procedure as in Example 1-1* was repeated except that 2,4,6-triisopropylbenzenesulfonyl chloride was used instead of 2,4,6-tricyclohexylbenzenesulfonyl chloride, and benzenesulfonyl chloride was used instead of p-toluenesulfonyl chloride. There was synthesized 10-phenylphenoxathiinium 2,4,6-triisopropyl-N-(benzenesulfonyl)benzenesulfonamidate, designated Salt-2.

### Example 1-3*

### Synthesis of Salt-3

The same procedure as in Example 1-1* was repeated except that 2,5-dimethylbenzenesulfonyl chloride was used instead of 2,4,6-tricyclohexylbenzenesulfonyl chloride, methanesulfonyl chloride was used instead of p-toluenesulfonyl chloride, and a triphenylsulfonium chloride aqueous solution was used instead of the 10-phenylphenoxathiinium chloride aqueous solution. There was synthesized triphenylsulfonium 2,5-dimethyl-N-(methanesulfonyl)benzenesulfonamidate, designated Salt-3.

### Example 1-4*

### Synthesis of Salt-4

The same procedure as in Example 1-1* was repeated except that 2,4,6-triisopropylbenzenesulfonyl chloride was used instead of 2,4,6-tricyclohexylbenzenesulfonyl chloride, trifluoromethanesulfonyl chloride was used instead of p-toluenesulfonyl chloride, and a triphenylsulfonium chloride aqueous solution was used instead of the 10-phenylphenoxathiinium chloride aqueous solution. There was synthesized triphenylsulfonium 2,4,6-triisopropyl-N-(trifluoromethanesulfonyl)-benzenesulfonamidate, designated Salt-4.

### Example 1-5*

### Synthesis of Salt-5

Methanesulfonamide, 14.3 g, was dissolved in 100 g of water, 25.1 g of 10-camphorsulfonyl chloride was added thereto, and 8.4 g of potassium hydroxide was added thereto. The mixture was stirred at room temperature for 3 hours, whereupon 1,000 g of methylene chloride was added. Then triphenylsulfonium chloride aqueous solution was added to the solution, which was stirred for 30 minutes. The organic layer was taken out, washed twice with 300 g of water, concentrated, and purified by chromatography. There was obtained 22.4 g (yield 40%) of the target compound, designated Salt-5.

### [2] Synthesis of polymers

### Synthesis Example 1*

### Synthesis of Polymer 1

A 3-L flask was charged with 314.4 g of 4-acetoxystyrene, 22.0 g of 4-chlorostyrene, 190.7 g of indene, and 675 g of toluene as solvent. The reactor was cooled at -70°C in nitrogen atmosphere, after which vacuum pumping and nitrogen flow were repeated three times. The reactor was warmed up to room temperature, whereupon 40.5 g of 2,2'-azobis(2,4-dimethylvaleronitrile) (V-65 by Wako Pure Chemical Industries, Ltd.) was added as polymerization initiator. The reactor was heated at 45°C, whereupon reaction ran for 20 hours. The reactor was further heated at 55°C whereupon reaction ran for further 20 hours. The reaction solution was concentrated to 1/2 in volume. The concentrate was poured to 15.0 L of methanol for precipitation. A white solid was collected by filtration and vacuum dried at 40°C, obtaining 309 g of a white polymer.

The polymer was again dissolved in a mixture of 488 g of methanol and 540 g of THF. 162 g of triethylamine and 32 g of water were added to the solution, whereupon deprotection reaction was carried out at 60°C for 40 hours. The reaction solution was concentrated and dissolved in 870 g of ethyl acetate. The solution was subjected to once neutralization/separation/washing with a mixture of 250 g of water and 98 g of acetic acid, once separation/washing with a mixture of 225 g of water and 75 g of pyridine, and 4 times separation/washing with 225 g of water. The upper layer, ethyl acetate solution was concentrated, dissolved in 250 g of acetone, and poured into 15 L of water for precipitation. The precipitate was filtered and vacuum dried at 50°C for 40 hours, obtaining 223 g of a white polymer.

The polymer was analyzed by ¹³C-NMR, ¹H-NMR and GPC, with the results shown below.

| | | | |
|---|---|---|---|
| | 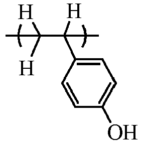 | 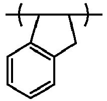 | 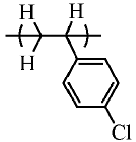 |
| Copolymer compositional ratio | 80.0 | 10.0 | 10.0 |

Mw = 4,500
Mw/Mn = 1.65

### Synthesis Example 2*

### Synthesis of Polymer 7

In nitrogen atmosphere, 890 g of 50.0 wt% PGMEA solution of 4-hydroxystyrene, 47.7 g of acenaphthylene, 310 g of 54.7 wt% PGMEA solution of 4-(2-hydroxy-2-propyl)-styrene, 87.0 g of triphenylsulfonium 1,1,3,3,3-pentafluoro-2-methacryloyloxypropane-1-sulfonate, 96.1 g of dimethyl 2,2'-azobis(2-methylpropionate) (V-601 by Wako Pure Chemical Industries, Ltd.), and 360 g of γ-butyrolactone and 220 g of PGMEA as solvent were fed into a 3000-mL dropping cylinder to form a monomer solution. In nitrogen atmosphere, a 5000-mL polymerization flask was charged with 580 g of γ-butyrolactone, which was heated at 80°C. The monomer solution was added dropwise from the dropping cylinder to the flask over 4 hours. After the completion of dropwise addition, the polymerization solution was continuously stirred for 18 hours while maintaining its temperature at 80°C. The polymerization solution was cooled to room temperature and added dropwise to 22.5 kg of diisopropyl ether whereupon the copolymer agglomerated. Diisopropyl ether was decanted off, and the copolymer was dissolved in 2,250 g of acetone. The acetone solution was added dropwise to 22.5 kg of diisopropyl ether whereupon the copolymer precipitated. The copolymer precipitate was collected by filtration and dissolved in 2,250 g of acetone again. The acetone solution was added dropwise to 22.5 kg of water. The copolymer precipitate was collected by filtration and dried at 40°C for 40 hours, obtaining 700 g of a white polymer.

The polymer designated Polymer 7 was analyzed by ¹³C-NMR, ¹H-NMR and GPC, with the results shown below.

| | | | | |
|---|---|---|---|---|
| | 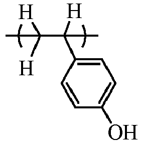 | 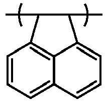 | 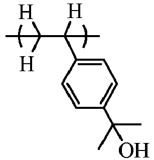 | 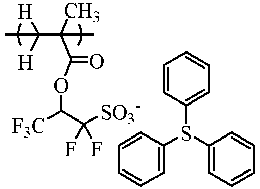 |
| Copolymer compositional ratio | 66.0 | 9.0 | 21.5 | 3.5 |

Mw = 13,000
Mw/Mn = 1.62

### Synthesis Examples 3 to 13*

### Synthesis of Polymers 2 to 6, 8 to 13

Polymers 2 to 6 were synthesized by the same procedures as in Synthesis Example 1* except that the type and amount (molar ratio) of monomers were changed. Polymers 8 to 13 were synthesized by the same procedures as in Synthesis Example 2 except that the type and amount (molar ratio) of monomers were changed.

For Polymers 1 to 13, the type and molar ratio of monomers are tabulated in Table 1. The structures of recurring units incorporated in the polymers are shown in Tables 2 to 5.

**Table 1**

| | Unit 1 | Ratio (mol%) | Unit 2 | Ratio (mol%) | Unit 3 | Ratio (mol%) | Unit 4 | Ratio (mol%) | Mw | Mw/Mn |
|---|---|---|---|---|---|---|---|---|---|---|
| Polymer 1 | A-1 | 80.0 | B-1 | 10.0 | B-4 | 10.0 | | | 4,500 | 1.65 |
| Polymer 2 | A-1 | 80.0 | B-2 | 8.0 | B-3 | 12.0 | | | 4,200 | 1.64 |
| Polymer 3 | A-1 | 60.0 | B-2 | 10.0 | C-1 | 30.0 | | | 4,300 | 1.62 |
| Polymer 4 | A-1 | 35.0 | B-2 | 10.0 | C-2 | 55.0 | | | 3,800 | 1.61 |
| Polymer 5 | A-1 | 45.0 | B-2 | 10.0 | C-3 | 45.0 | | | 4,100 | 1.63 |
| Polymer 6 | A-1 | 55.0 | B-3 | 15.0 | C-1 | 30.0 | | | 4,000 | 1.62 |
| Polymer 7 | A-1 | 66.0 | B-2 | 9.0 | C-1 | 21.5 | P-1 | 3.5 | 13,000 | 1.62 |
| Polymer 8 | A-1 | 71.0 | B-2 | 10.0 | C-1 | 15.0 | P-2 | 4.0 | 14,000 | 1.65 |
| Polymer 9 | A-1 | 68.0 | B-2 | 9.0 | C-1 | 20.0 | P-3 | 3.0 | 14,000 | 1.64 |
| Polymer 10 | A-1 | 66.0 | B-2 | 10.0 | C-1 | 21.0 | P-4 | 3.0 | 16,000 | 1.63 |
| Polymer 11 | A-1 | 52.0 | B-2 | 10.0 | C-2 | 34.5 | P-1 | 3.5 | 16,000 | 1.63 |
| Polymer 12 | A-1 | 56.5 | B-2 | 9.0 | C-3 | 31.0 | P-1 | 3.5 | 15,000 | 1.62 |
| Polymer 13 | A-2 | 60.0 | B-3 | 9.0 | C-1 | 27.5 | P-1 | 3.5 | 16,000 | 1.62 |

**Table 2**

| A-1 | A-2 |
|---|---|
| 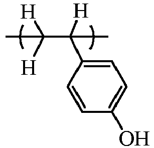 | 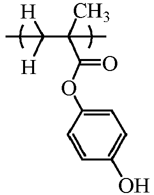 |

**Table 3**

| B-1 | B-2 | B-3 | B-4 |
|---|---|---|---|
| 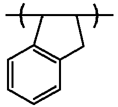 | 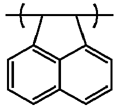 | 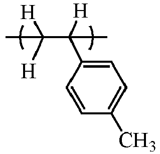 | 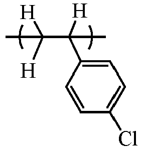 |

**Table 4**

| C-1 | C-2 | C-3 |
|---|---|---|
| 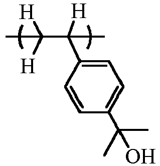 | 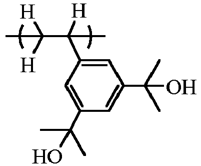 | 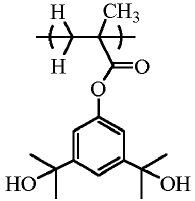 |

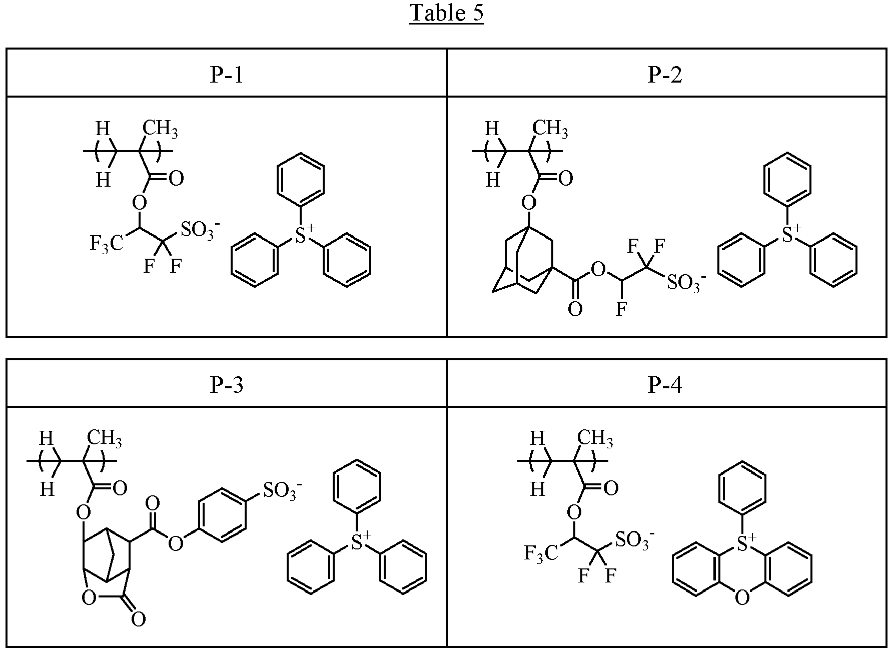

### [3] Preparation of negative resist compositions

### Examples 2-1 to 2-23 and Comparative Examples 1-1 to 1-7

Negative resist compositions in solution form were prepared by dissolving a polymer (Polymers 1 to 13), an acid generator (PAG-1 to PAG-4), a onium salt (Salt-1 to Salt-5) or comparative quencher (Q-1 to Q-3) as quencher, and tetramethoxymethylglycoluril (TMGU) as crosslinker in an organic solvent in accordance with the formulation shown in Tables 6 and 7, and filtering through a filter with a pore size of 0.2 µm or a nylon or UPE filter with a pore size of 0.02 µm. The solvents used were propylene glycol monomethyl ether acetate (PGMEA), ethyl lactate (EL), and propylene glycol monomethyl ether (PGME). All the compositions contained 0.075 pbw of surfactant PF-636 (Omnova Solutions).

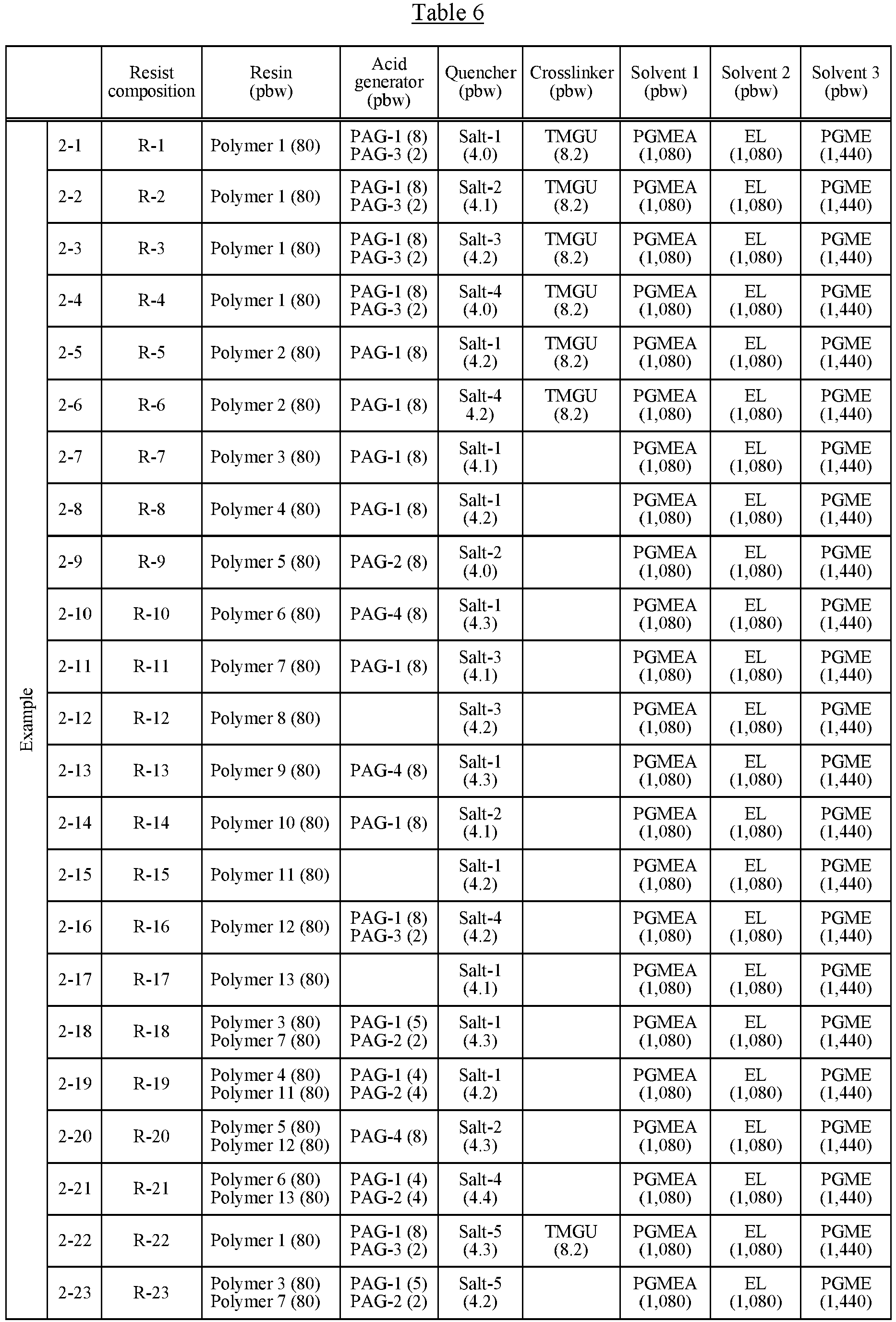

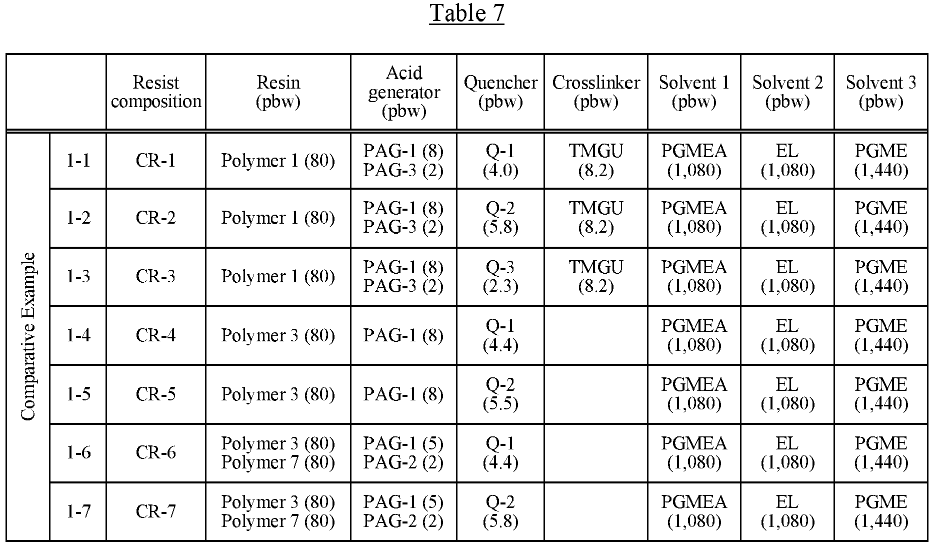

In Tables 6 and 7, Salt-1 to Salt-5, Q-1 to Q-3, and PAG-1 to PAG-4 have the following structure.

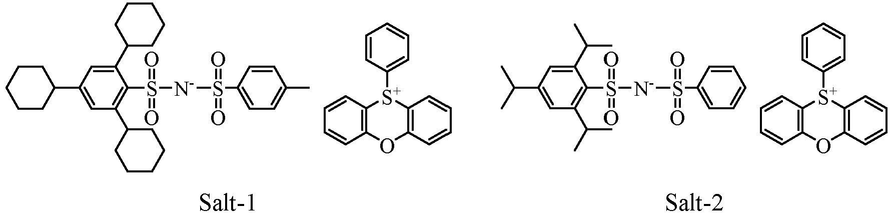

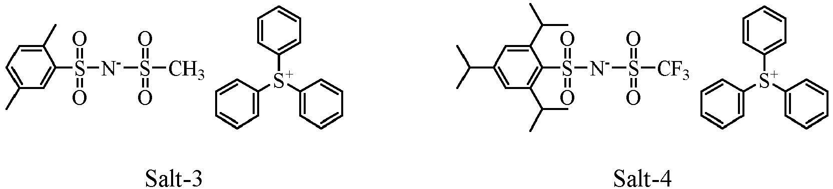

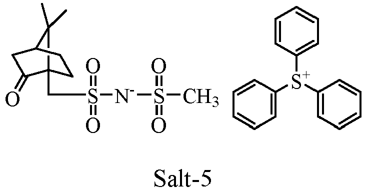

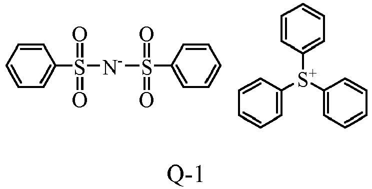

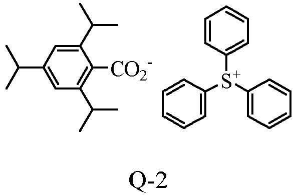

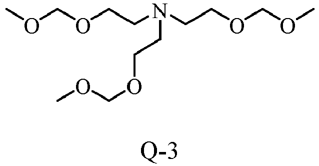

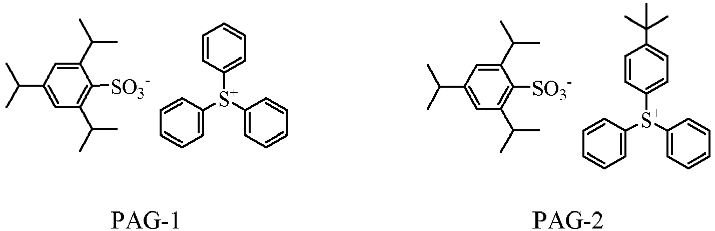

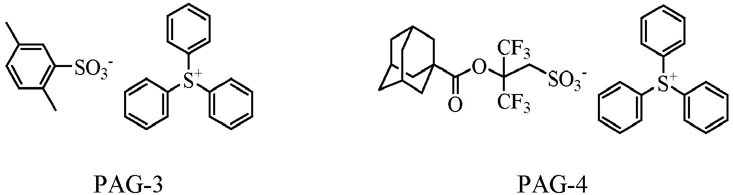

### [4] EB writing test

### Examples 3-1 to 3-23 and Comparative Examples 2-1 to 2-7

Using a coater/developer system ACT-M (Tokyo Electron Ltd.), each of the negative resist compositions (R-1 to R-23 and CR-1 to CR-7) was spin coated on a mask blank of 152 mm squares having a chromium oxynitride film at the outermost surface, and prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

The coated mask blanks were exposed to electron beam using an EB writer system EBM-5000Plus (NuFlare Technology Inc., accelerating voltage 50 kV), then baked (PEB) at 120°C for 600 seconds, and developed in a 2.38 wt% tetramethylammonium hydroxide (TMAH) aqueous solution, thereby yielding negative patterns.

The patterned mask blank was observed under a top-down scanning electron microscope (TDSEM). The optimum dose (Eop) was defined as the exposure dose (µC/cm²) which provided a 1:1 resolution at the top and bottom of a 400-nm 1:1 line-and-space pattern. The LS resolution (or maximum resolution) was defined as the minimum size of a pattern that could be resolved at the exposure dose capable of resolving a 400-nm LS pattern at 1:1. The LER of a 200-nm L/S pattern was measured under SEM. The maximum resolutions (IL resolution and IS resolution) of an isolated line (IL) and an isolated space (IS) were also measured. Notably, IL is the resolution of an isolated single line feature, and IS is the resolution of an isolated single space feature. The pattern was visually observed to judge whether or not the pattern profile was rectangular.

The test results are shown in Table 8.

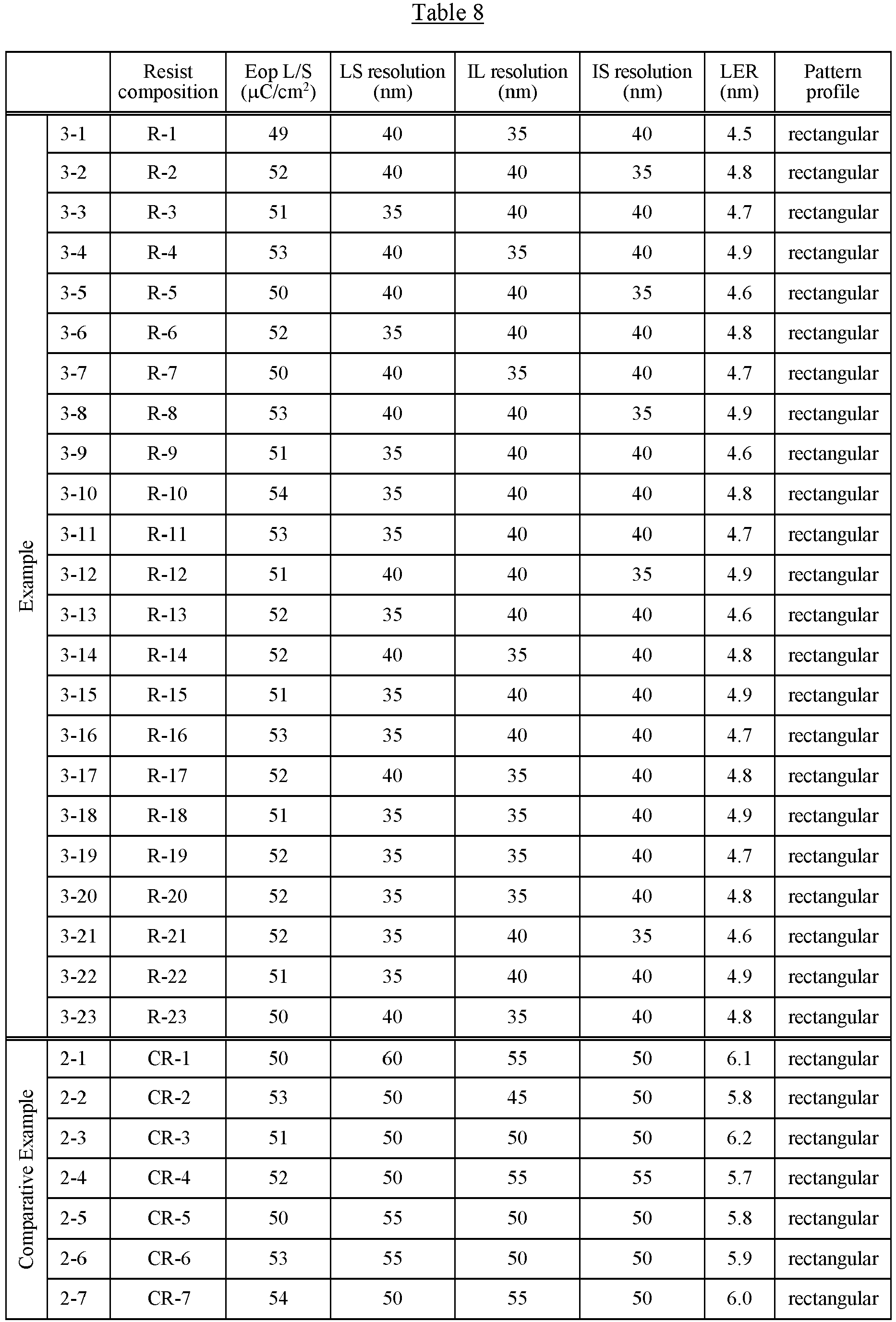

All the inventive resist compositions (Examples 3-1 to 3-23) comprising the onium salts show satisfactory results with respect to resolution, rectangular pattern profile, and LER. The resist compositions of Comparative Examples 2-1 to 2-7 are inferior in resolution and LER to Examples.

It has been demonstrated that by using the inventive resist composition to form a resist film and exposing it via high-energy radiation lithography, a pattern having high resolution and a minimal LER is formed. The resist composition forms a pattern without defects and development residues while avoiding chemical flare and local heat during high current writing. The resist pattern forming process using the inventive resist composition is useful in the photolithography for the fabrication of microelectronic devices, especially processing of photomask blanks.

Although some preferred embodiments have been described, many modifications and variations may be made thereto in light of the above teachings. It is therefore to be understood that the invention may be practiced otherwise than as specifically described without departing from the scope of the appended claims.

## Claims

1. A negative resist composition comprising an onium salt having the formula (A): 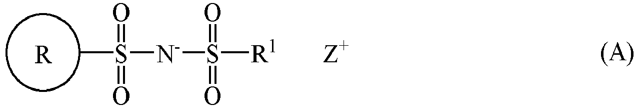 wherein R¹ is a C₁-C₂₀ monovalent hydrocarbon group which may contain a heteroatom,
the ring R is a group having the formula (A1), or a C₆-C₃₀ group having an alicyclic structure in which some carbon may be replaced by a moiety containing sulfur, oxygen or nitrogen, 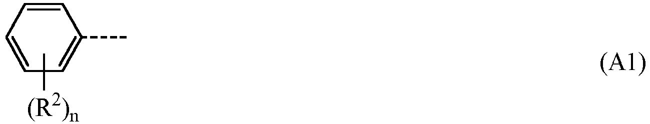 wherein the broken line designates a valence bond, R² is each independently a C₁-C₂₀ monovalent hydrocarbon group which may contain a heteroatom, n is an integer of 2 to 5,
Z⁺ is a sulfonium cation having the formula (A2) or iodonium cation having the formula (A3): 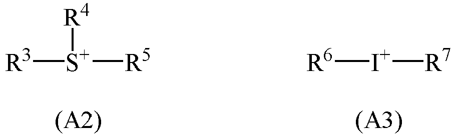 wherein R³ to R⁷ are each independently a C₁-C₂₀ monovalent hydrocarbon group which may contain a heteroatom, any two or more of R³, R⁴ and R⁵ may bond together to form a ring with the sulfur atom to which they are attached; and
(B) a base polymer containing a polymer comprising recurring units having the formula (B1): 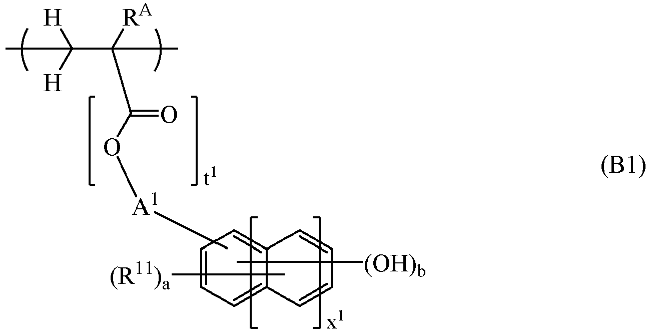 wherein R^{A} is hydrogen, fluorine, methyl or trifluoromethyl, R¹¹ is each independently halogen, an optionally halogenated C₂-C₈ acyloxy group, optionally halogenated C₁-C₆ alkyl group, or optionally halogenated C₁-C₆ alkoxy group, A¹ is a single bond or C₁-C₁₀ alkanediyl group in which an ether bond may intervene in a carbon-carbon bond, t¹ is 0 or 1, x¹ is an integer of 0 to 2, a is an integer satisfying 0 ≤ a ≤ 5+2x¹-b, and b is an integer of 1 to 5.

2. The negative resist composition of claim 1 wherein the ring R is a group having the formula (A1).

3. The negative resist composition of claim 2 wherein R² is a C₃-C₂₀ branched or cyclic monovalent hydrocarbon group.

4. The negative resist composition of claim 2 or 3 wherein at least one R² is bonded at the ortho-position relative to the carbon atom bonded to the sulfonyl group.

5. The negative resist composition of any one of claims 2 to 4 wherein n is an integer of 3 to 5.

6. The negative resist composition of any one of claims 1 to 5 wherein the polymer further comprises recurring units of at least one type selected from recurring units having the formulae (B2), (B3) and (B4): 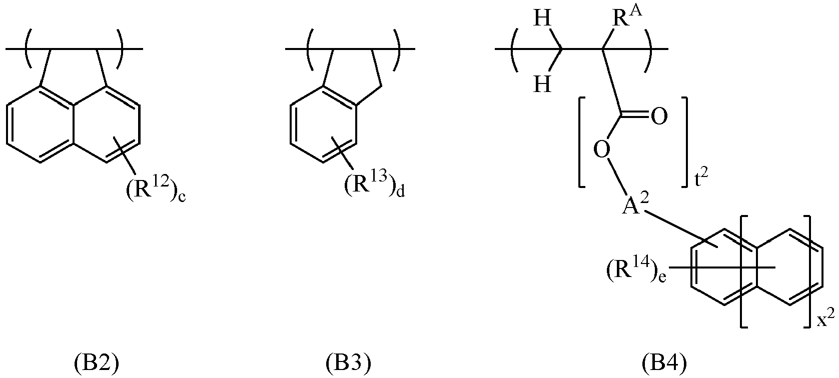
wherein R^{A} is hydrogen, fluorine, methyl or trifluoromethyl,
R¹² and R¹³ are each independently hydroxyl, halogen, acetoxy, an optionally halogenated C₁-C₈ alkyl group, optionally halogenated C₁-C₈ primary alkoxy group, C₂-C₈ secondary alkoxy group, optionally halogenated C₂-C₈ acyloxy group, or optionally halogenated C₂-C₈ alkylcarbonyloxy group,
R¹⁴ is an acetyl group, acetoxy group, C₁-C₂₀ alkyl group, C₁-C₂₀ primary alkoxy group, C₂-C₂₀ secondary alkoxy group, C₂-C₂₀ acyloxy group, C₂-C₂₀ alkoxyalkyl group, C₂-C₂₀ alkylthioalkyl group, halogen atom, nitro group, or cyano group,
A² is a single bond or C₁-C₁₀ alkanediyl group in which an ether bond may intervene in a carbon-carbon bond,
c and d are each independently an integer of 0 to 4, e is an integer of 0 to 5, x² is an integer of 0 to 2, and t² is 0 or 1.

7. The negative resist composition of any one of claims 1 to 6 wherein the polymer further comprises recurring units having the formula (B5): 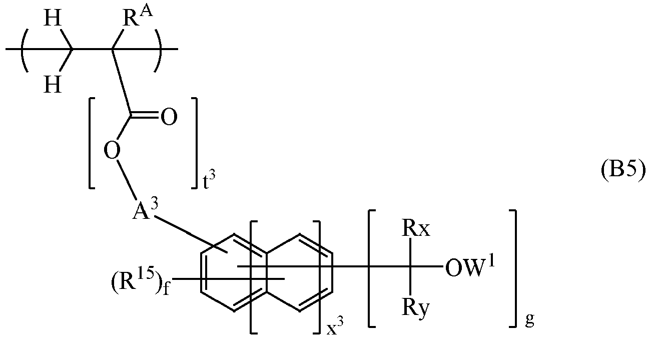
wherein R^{A} is hydrogen, fluorine, methyl or trifluoromethyl,
R¹⁵ is each independently halogen, an optionally halogenated C₂-C₈ acyloxy group, optionally halogenated C₁-C₆ alkyl group, or optionally halogenated C₁-C₆ alkoxy group,
A³ is a single bond or C₁-C₁₀ alkanediyl group in which an ether bond may intervene in a carbon-carbon bond,
W¹ is hydrogen, a C₁-C₁₀ monovalent aliphatic hydrocarbon group in which an ether bond, carbonyl moiety or carbonyloxy moiety may intervene in a carbon-carbon bond, or an optionally substituted monovalent aromatic ring group,
Rx and Ry are each independently hydrogen, an optionally hydroxy or alkoxy-substituted C₁-C₁₅ alkyl group or an optionally substituted monovalent aromatic ring group, with the proviso that both Rx and Ry are not hydrogen at the same time, Rx and Ry may bond together to form a ring with the carbon atom to which they are attached,
x³ is an integer of 0 to 2, t³ is 0 or 1, f is an integer satisfying 0 ≤ f ≤ 5+2x³-g, and g is an integer of 1 to 3.

8. The negative resist composition of claim 7 wherein the polymer further comprises recurring units of at least one type selected from recurring units having the formulae (B6), (B7) and (B8): 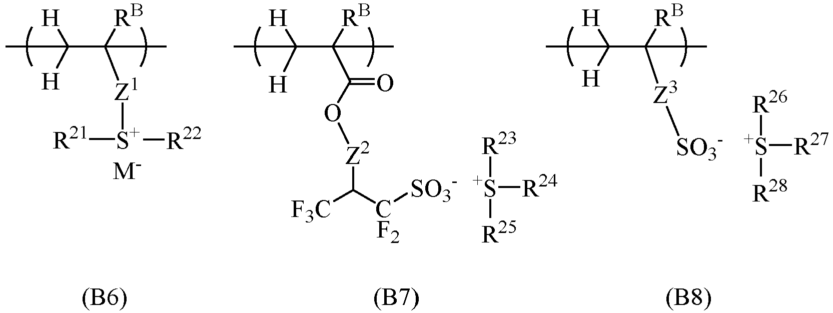
wherein R^{B} is each independently hydrogen or methyl,
Z¹ is a single bond, phenylene group, -O-Z¹¹-, -C(=O)-O-Z¹¹-, or -C(=O)-NH-Z¹¹-, Z¹¹ is a C₁-C₆ alkanediyl, C₂-C₆ alkenediyl or phenylene group, which may contain a carbonyl moiety, ester bond, ether bond or hydroxyl moiety,
Z² is a single bond or -Z²¹-C(=O)-O-, Z²¹ is a C₁-C₂₀ divalent hydrocarbon group which may contain a heteroatom,
Z³ is a single bond, methylene, ethylene, phenylene, fluorinated phenylene, -O-Z³¹-, -C(=O)-O-Z³¹-, or -C(=O)-NH-Z³¹-, Z³¹ is a C₁-C₆ alkanediyl, C₂-C₆ alkenediyl or phenylene group, which may contain a carbonyl moiety, ester bond, ether bond or hydroxyl moiety,
R²¹ to R²⁸ are each independently a C₁-C₂₀ monovalent hydrocarbon group which may contain a heteroatom, or R²¹ and R²² may bond together to form a ring with the sulfur atom to which they are attached, or any two or more of R²³, R²⁴ and R²⁵ may bond together to form a ring with the sulfur atom to which they are attached, any two or more of R²⁶, R²⁷ and R²⁸ may bond together to form a ring with the sulfur atom to which they are attached, and
M⁻ is a non-nucleophilic counter ion.

9. The negative resist composition of any one of claims 1 to 8 wherein the polymer comprises recurring units having the formula (B1-1), recurring units having the formula (B5-1), and recurring units having the formula (B7): 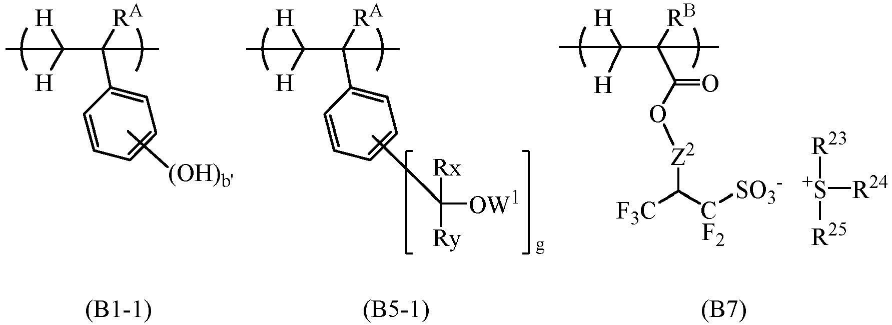
wherein R^{A} is each independently hydrogen, fluorine, methyl or trifluoromethyl,
R^{B} is hydrogen or methyl,
W¹ is hydrogen, a C₁-C₁₀ monovalent aliphatic hydrocarbon group in which an ether bond, carbonyl moiety or carbonyloxy moiety may intervene in a carbon-carbon bond, or an optionally substituted monovalent aromatic ring group,
Rx and Ry are each independently hydrogen, an optionally hydroxy or alkoxy-substituted C₁-C₁₅ alkyl group or an optionally substituted monovalent aromatic ring group, with the proviso that both Rx and Ry are not hydrogen at the same time, Rx and Ry may bond together to form a ring with the carbon atom to which they are attached,
Z² is a single bond or -Z²¹-C(=O)-O-, Z²¹ is a C₁-C₂₀ divalent hydrocarbon group which may contain a heteroatom,
R²³ to R²⁵ are each independently a C₁-C₂₀ monovalent hydrocarbon group which may contain a heteroatom, or any two or more of R²³, R²⁴ and R²⁵ may bond together to form a ring with the sulfur atom to which they are attached,
b' is an integer of 1 to 3, and g is an integer of 1 to 3.

10. The negative resist composition of claim 8 or 9 wherein the base polymer (B) further contains a polymer free of recurring units having the formula (B6), recurring units having the formula (B7), and recurring units having the formula (B8).

11. The negative resist composition of any one of claims 1 to 10, further comprising (C) a crosslinker.

12. The negative resist composition of claim 7, which is free of a crosslinker.

13. The negative resist composition of any one of claims 1 to 12, further comprising (E) an acid generator.

14. A photomask blank coated with the negative resist composition of any one of claims 1 to 13.

15. A resist pattern forming process comprising the steps of:
applying the negative resist composition of any one of claims 1 to 13 onto a substrate to form a resist film thereon,
exposing the resist film patternwise to high-energy radiation, and
developing the resist film in an alkaline developer to form a resist pattern.

16. The process of claim 15 wherein the high-energy radiation is EUV or EB.

17. The process of claim 15 or 16 wherein the substrate is a photomask blank.

## Patentansprüche

1. Negative Resistzusammensetzung, umfassend ein Oniumsalz, das die Formel (A) aufweist: 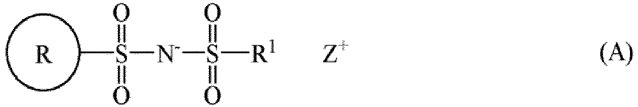 wobei R¹ eine monovalente C₁-C₂₀-Kohlenwasserstoffgruppe ist, die ein Heteroatom enthalten kann,
der Ring R eine Gruppe ist, die die Formel (A1) aufweist, oder eine C₆-C₃₀-Gruppe ist, die eine alicyclische Struktur aufweist, in der etwas Kohlenstoff durch einen Rest ersetzt sein kann, der Schwefel, Sauerstoff oder Stickstoff enthält, 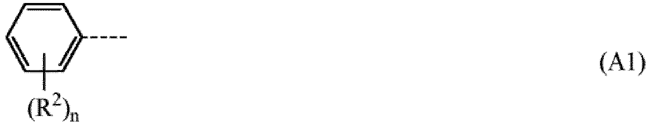 wobei die gestrichelte Linie eine Valenzbindung bezeichnet, R² jeweils unabhängig eine monovalente C₁-C₂₀-Kohlenwasserstoffgruppe ist, die ein Heteroatom enthalten kann, n eine ganze Zahl von 2 bis 5 ist,
Z⁺ ein Sulfoniumkation ist, das die Formel (A2) aufweist, oder ein Iodoniumkation ist, das die Formel (A3) aufweist: 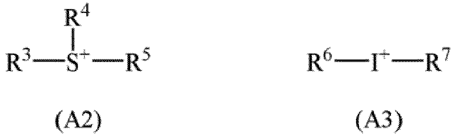 wobei R³ bis R⁷ jeweils unabhängig eine monovalente C₁-C₂₀-Kohlenwasserstoffgruppe sind, die ein Heteroatom enthalten kann, beliebige zwei oder mehr von R³, R⁴ und R⁵ aneinanderbinden können, um einen Ring mit dem Schwefelatom zu bilden, an dem sie angebracht sind; und
(B) ein Basispolymer, das ein Polymer enthält, das wiederkehrende Einheiten umfasst, die die Formel (B1) aufweisen: 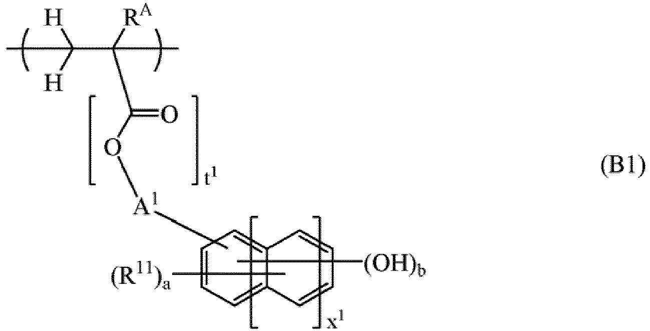 wobei R^{A} Wasserstoff, Fluor, Methyl oder Trifluormethyl ist, R¹¹ jeweils unabhängig Halogen, eine optional halogenierte C₂-C₈-Acyloxygruppe, optional halogenierte C₁-C₆-Alkylgruppe oder optional halogenierte C₁-C₆-Alkoxygruppe ist, A¹ eine Einfachbindung oder C₁-C₁₀-Alkandiylgruppe ist, in der eine Etherbindung in eine Kohlenstoff-Kohlenstoff-Bindung eingreifen kann, t¹ 0 oder 1 ist, x¹ eine ganze Zahl von 0 bis 2 ist, a eine ganze Zahl ist, die 0 ≤ a ≤ 5+2x¹-b erfüllt, und b eine ganze Zahl von 1 bis 5 ist.

2. Negative Resistzusammensetzung nach Anspruch 1, wobei der Ring R eine Gruppe ist, die die Formel (A1) aufweist.

3. Negative Resistzusammensetzung nach Anspruch 2, wobei R² eine verzweigte oder cyclische monovalente C₃-C₂₀-Kohlenwasserstoffgruppe ist.

4. Negative Resistzusammensetzung nach Anspruch 2 oder 3, wobei mindestens ein R² an der ortho-Position in Bezug auf das an die Sulfonylgruppe gebundene Kohlenstoffatom gebunden ist.

5. Negative Resistzusammensetzung nach einem der Ansprüche 2 bis 4, wobei n eine ganze Zahl von 3 bis 5 ist.

6. Negative Resistzusammensetzung nach einem der Ansprüche 1 bis 5, wobei das Polymer ferner wiederkehrende Einheiten mindestens eines Typs umfasst, die aus wiederkehrenden Einheiten ausgewählt sind, die die Formeln (B2), (B3) und (B4) aufweisen: 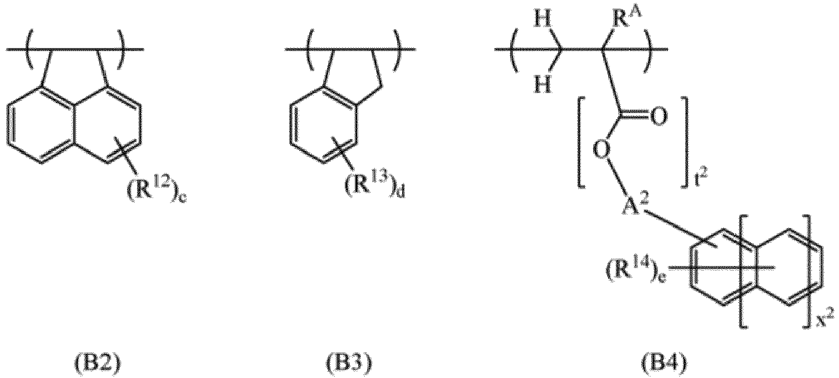
wobei R^{A} Wasserstoff, Fluor, Methyl oder Trifluormethyl ist,
R¹² und R¹³ jeweils unabhängig Hydroxyl, Halogen, Acetoxy, eine optional halogenierte C₁-C₈-Alkylgruppe, optional halogenierte primäre C₁-C₈-Alkoxygruppe, sekundäre C₂-C₈-Alkoxygruppe, optional halogenierte C₂-C₈-Acyloxygruppe oder optional halogenierte C₂-C₈-Alkylcarbonyloxygruppe sind,
R¹⁴ eine Acetylgruppe, Acetoxygruppe, C₁-C₂₀-Alkylgruppe, primäre C₁-C₂₀-Alkoxygruppe, sekundäre C₂-C₂₀-Alkoxygruppe, C₂-C₂₀-Acyloxygruppe, C₂-C₂₀-Alkoxyalkylgruppe, C₂-C₂₀-Alkylthioalkylgruppe, ein Halogenatom, eine Nitrogruppe oder Cyanogruppe ist,
A² eine Einfachbindung oder C₁-C₁₀-Alkandiylgruppe ist, in der eine Etherbindung in eine Kohlenstoff-Kohlenstoff-Bindung eingreifen kann,
c und d jeweils unabhängig eine ganze Zahl von 0 bis 4 sind, e eine ganze Zahl von 0 bis 5 ist, x² eine ganze Zahl von 0 bis 2 ist und t² 0 oder 1 ist.

7. Negative Resistzusammensetzung nach einem der Ansprüche 1 bis 6, wobei das Polymer ferner wiederkehrende Einheiten umfasst, die die Formel (B5) aufweisen: 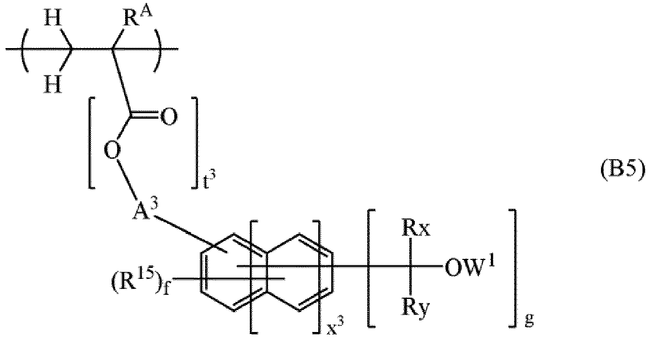
wobei R^{A} Wasserstoff, Fluor, Methyl oder Trifluormethyl ist,
R¹⁵ jeweils unabhängig Halogen, eine optional halogenierte C₂-C₈-Acyloxygruppe, optional halogenierte C₁-C₆-Alkylgruppe oder optional halogenierte C₁-C₆-Alkoxygruppe ist,
A³ eine Einfachbindung oder C₁-C₁₀-Alkandiylgruppe ist, in der eine Etherbindung in eine Kohlenstoff-Kohlenstoff-Bindung eingreifen kann,
W¹ Wasserstoff, eine monovalente aliphatische C₁-C₁₀-Kohlenwasserstoffgruppe, in der eine Etherbindung, ein Carbonylrest oder ein Carbonyloxyrest in eine Kohlenstoff-Kohlenstoff-Bindung eingreifen kann, oder eine optional substituierte monovalente aromatische Ringgruppe ist,
Rx und Ry jeweils unabhängig Wasserstoff, eine optional hydroxy- oder alkoxysubstituierte C₁-C₁₅-Alkylgruppe oder eine optional substituierte monovalente aromatische Ringgruppe sind, mit der Maßgabe, dass sowohl Rx als auch Ry nicht gleichzeitig Wasserstoff sind, Rx und Ry aneinanderbinden können, um einen Ring mit dem Kohlenstoffatom zu bilden, an dem sie angebracht sind,
x³ eine ganze Zahl von 0 bis 2 ist, t³ 0 oder 1 ist, f eine ganze Zahl ist, die 0 ≤ f ≤ 5+2x³-g erfüllt, und g eine ganze Zahl von 1 bis 3 ist.

8. Negative Resistzusammensetzung nach Anspruch 7, wobei das Polymer ferner wiederkehrende Einheiten mindestens eines Typs umfasst, die aus wiederkehrenden Einheiten ausgewählt sind, die die Formeln (B6), (B7) und (B8) aufweisen: 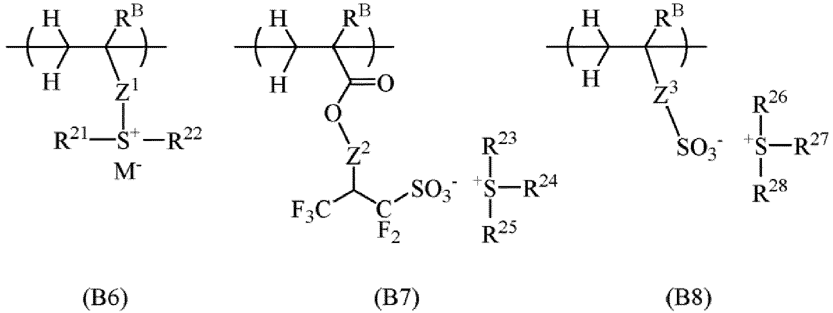
wobei R^{B} jeweils unabhängig Wasserstoff oder Methyl ist,
Z¹ eine Einfachbindung, Phenylengruppe, -O-Z¹¹-, -C(=O)-O-Z¹¹- oder -C(=O)-NH-Z¹¹- ist, Z¹¹ eine C₁-C₆-Alkandiyl-, C₂-C₆-Alkendiyl- oder Phenylengruppe ist, die einen Carbonylrest, eine Esterbindung, eine Etherbindung oder einen Hydroxylrest enthalten kann,
Z² eine Einfachbindung oder -Z²¹-C(=O)-O- ist, Z²¹ eine divalente C₁-C₂₀-Kohlenwasserstoffgruppe ist, die ein Heteroatom enthalten kann,
Z³ eine Einfachbindung, Methylen, Ethylen, Phenylen, fluoriertes Phenylen, -O-Z³¹-, - C(=O)-O-Z³¹- oder -C(=O)-NH-Z³¹- ist, Z³¹ eine C₁-C₆-Alkandiyl-, C₂-C₆-Alkendiyl- oder Phenylengruppe ist, die einen Carbonylrest, eine Esterbindung, eine Etherbindung oder einen Hydroxylrest enthalten kann,
R²¹ bis R²⁸ jeweils unabhängig eine monovalente C₁-C₂₀-Kohlenwasserstoffgruppe sind, die ein Heteroatom enthalten kann, oder R²¹ und R²² aneinanderbinden können, um einen Ring mit dem Schwefelatom zu bilden, an dem sie angebracht sind, oder beliebige zwei oder mehr von R²³, R²⁴ und R²⁵ aneinanderbinden können, um einen Ring mit dem Schwefelatom zu bilden, an dem sie angebracht sind, beliebige zwei oder mehr von R²⁶, R²⁷ und R²⁸ aneinanderbinden können, um einen Ring mit dem Schwefelatom zu bilden, an dem sie angebracht sind, und
M⁻ ein nicht nucleophiles Gegenion ist.

9. Negative Resistzusammensetzung nach einem der Ansprüche 1 bis 8, wobei das Polymer wiederkehrende Einheiten, die die Formel (B1-1) aufweisen, wiederkehrende Einheiten, die die Formel (B5-1) aufweisen, und wiederkehrende Einheiten, die die Formel (B7) aufweisen, umfasst: 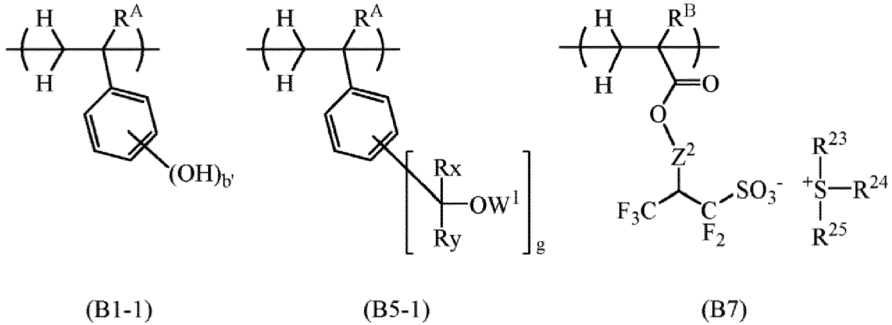
wobei R^{A} jeweils unabhängig Wasserstoff, Fluor, Methyl oder Trifluormethyl ist,
R^{B} Wasserstoff oder Methyl ist,
W¹ Wasserstoff, eine monovalente aliphatische C₁-C₁₀-Kohlenwasserstoffgruppe, in der eine Etherbindung, ein Carbonylrest oder ein Carbonyloxyrest in eine Kohlenstoff-Kohlenstoff-Bindung eingreifen kann, oder eine optional substituierte monovalente aromatische Ringgruppe ist,
Rx und Ry jeweils unabhängig Wasserstoff, eine optional hydroxy- oder alkoxysubstituierte C₁-C₁₅-Alkylgruppe oder eine optional substituierte monovalente aromatische Ringgruppe sind, mit der Maßgabe, dass sowohl Rx als auch Ry nicht gleichzeitig Wasserstoff sind, Rx und Ry aneinanderbinden können, um einen Ring mit dem Kohlenstoffatom zu bilden, an dem sie angebracht sind,
Z² eine Einfachbindung oder -Z²¹-C(=O)-O- ist, Z²¹ eine divalente C₁-C₂₀-Kohlenwasserstoffgruppe ist, die ein Heteroatom enthalten kann,
R²³ bis R²⁵ jeweils unabhängig eine monovalente C₁-C₂₀-Kohlenwasserstoffgruppe sind, die ein Heteroatom enthalten kann, oder beliebige zwei oder mehr von R²³, R²⁴ und R²⁵ aneinanderbinden können, um einen Ring mit dem Schwefelatom zu bilden, an dem sie angebracht sind,
b' eine ganze Zahl von 1 bis 3 ist und g eine ganze Zahl von 1 bis 3 ist.

10. Negative Resistzusammensetzung nach einem der Ansprüche 8 oder 9, wobei das Basispolymer (B) ferner ein Polymer enthält, das frei von wiederkehrenden Einheiten, die die Formel (B6) aufweisen, wiederkehrenden Einheiten, die die Formel (B7) aufweisen, und wiederkehrenden Einheiten, die die Formel (B8) aufweisen, ist.

11. Negative Resistzusammensetzung nach einem der Ansprüche 1 bis 10, ferner umfassend (C) einen Crosslinker.

12. Negative Resistzusammensetzung nach Anspruch 7, die frei von einem Crosslinker ist.

13. Negative Resistzusammensetzung nach einem der Ansprüche 1 bis 12, ferner umfassend (E) einen Säuregenerator.

14. Fotomaskenrohling, der mit der negativen Resistzusammensetzung nach einem der Ansprüche 1 bis 13 beschichtet ist.

15. Resistmusterformungsverfahren, das die folgenden Schritte umfasst:
Auftragen der negativen Resistzusammensetzung nach einem der Ansprüche 1 bis 13 auf ein Substrat, um darauf einen Resistfilm zu formen,
Aussetzen des Resistfilms musterweise gegenüber Hochenergiestrahlung und
Entwickeln des Resistfilms in einem alkalischen Entwickler, um ein Resistmuster zu formen.

16. Verfahren nach Anspruch 15, wobei die Hochenergiestrahlung EUV oder EB ist.

17. Verfahren nach Anspruch 15 oder 16, wobei das Substrat ein Fotomaskenrohling ist.

## Revendications

1. Composition de réserve négative comprenant un sel d'onium ayant la formule (A) : 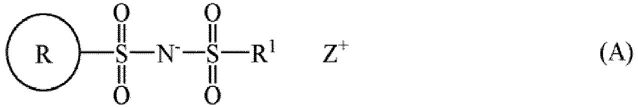 dans laquelle R¹ est un groupe hydrocarboné monovalent en C₁-C₂₀ qui peut contenir un hétéroatome,
le cycle R est un groupe ayant la formule (A1), ou un groupe en C₆-C₃₀ ayant une structure alicyclique dans laquelle du carbone peut être remplacé par un fragment contenant du soufre, de l'oxygène ou de l'azote, 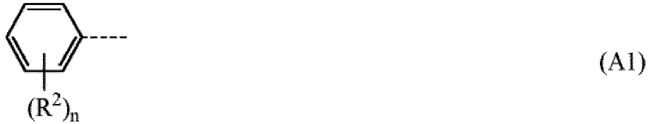 dans laquelle la ligne pointillée désigne une liaison de valence, R² est chacun indépendamment un groupe hydrocarboné monovalent en C₁-C₂₀ qui peut contenir un hétéroatome, n est un entier de 2 à 5,
Z⁺ est un cation sulfonium ayant la formule (A2) ou un cation iodonium ayant la formule (A3) : 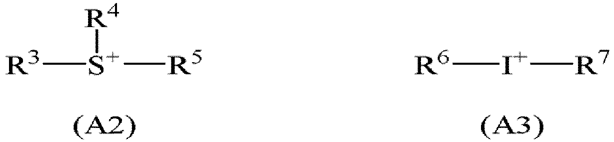 dans laquelle R³ à R⁷ sont chacun indépendamment un groupe hydrocarboné monovalent en C₁-C₂₀ qui peut contenir un hétéroatome, deux ou plus parmi R³, R⁴ et R⁵ peuvent se lier ensemble pour former un cycle avec l'atome de soufre auquel ils sont attachés ; et
(B) un polymère de base contenant un polymère comprenant des unités récurrentes ayant la formule (B1) : 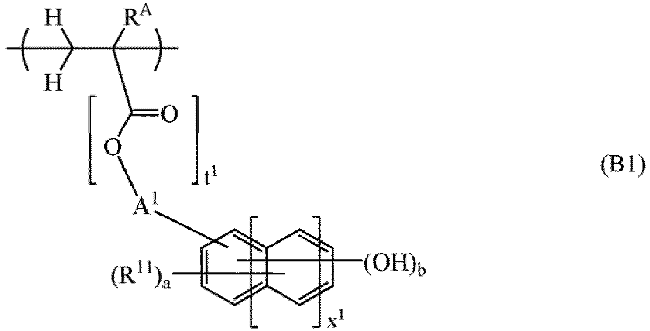 dans laquelle R^{A} est un groupe hydrogène, fluor, méthyle ou trifluorométhyle, R¹¹ est chacun indépendamment un groupe halogène, un groupe acyloxy en C₂-C₈ éventuellement halogéné, un groupe alkyle en C₁-C₆ éventuellement halogéné, ou un groupe alcoxy en C₁-C₆ éventuellement halogéné, A¹ est une liaison simple ou un groupe alcanediyle en C₁-C₁₀ dans lequel une liaison éther peut intervenir dans une liaison carbone-carbone, t¹ vaut 0 ou 1, x¹ est un entier de 0 à 2, a est un entier satisfaisant 0 ≤ a ≤ 5+2_{X¹}-b, et b est un entier de 1 à 5.

2. Composition de réserve négative selon la revendication 1, dans laquelle le cycle R est un groupe ayant la formule (A1).

3. Composition de réserve négative selon la revendication 2, dans laquelle R² est un groupe hydrocarboné monovalent ramifié ou cyclique en C₃-C₂₀.

4. Composition de réserve négative selon la revendication 2 ou 3, dans laquelle au moins un R² est lié en position ortho par rapport à l'atome de carbone lié au groupe sulfonyle.

5. Composition de réserve négative selon l'une quelconque des revendications 2 à 4, dans laquelle n est un entier de 3 à 5.

6. Composition de réserve négative selon l'une quelconque des revendications 1 à 5, dans laquelle le polymère comprend en outre des unités récurrentes d'au moins un type choisi parmi les unités récurrentes ayant les formules (B2), (B3) et (B4) : 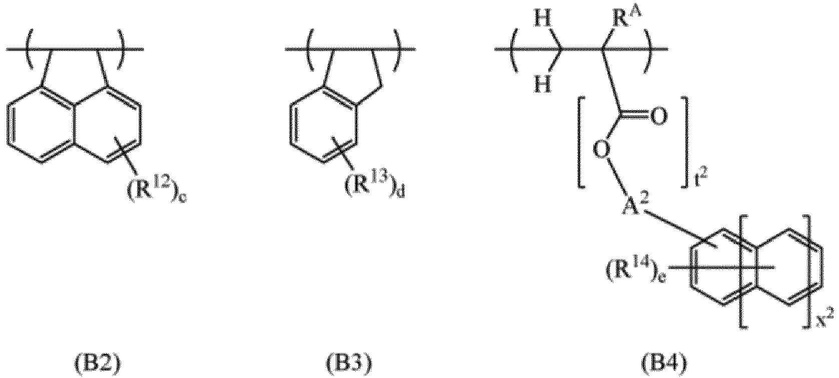
dans laquelle R^{A} est un groupe hydrogène, fluor, méthyle ou trifluorométhyle,
R¹² et R¹³ sont chacun indépendamment un groupe hydroxyle, halogène, acétoxy, un groupe alkyle en C₁-C₈ éventuellement halogéné, un groupe alcoxy primaire en C₁-C₈ éventuellement halogéné, un groupe alcoxy secondaire en C₂-C₈, un groupe acyloxy en C₂-C₈ éventuellement halogéné ou un groupe alkylcarbonyloxy en C₂-C₈ éventuellement halogéné,
R¹⁴ est un groupe acétyle, un groupe acétoxy, un groupe alkyle en C₁-C₂₀, un groupe alcoxy primaire en C₁-C₂₀, un groupe alcoxy secondaire en C₂-C₂₀, un groupe acyloxy en C₂-C₂₀, un groupe alcoxyalkyle en C₂-C₂₀, un groupe alkylthioalkyle en C₂-C₂₀, un atome d'halogène, un groupe nitro ou un groupe cyano,
A² est une liaison simple ou un groupe alcanediyle en C₁-C₁₀ dans lequel une liaison éther peut intervenir dans une liaison carbone-carbone,
c et d sont chacun indépendamment un entier de 0 à 4, e est un entier de 0 à 5, x² est un entier de 0 à 2 et t² vaut 0 ou 1.

7. Composition de réserve négative selon l'une quelconque des revendications 1 à 6, dans laquelle le polymère comprend en outre des unités récurrentes ayant la formule (B5) : 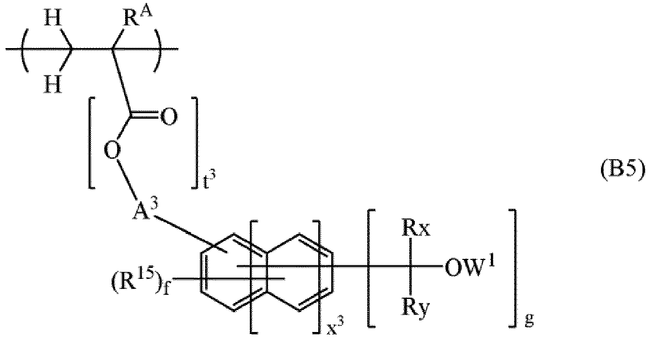
dans laquelle R^{A} est un groupe hydrogène, fluor, méthyle ou trifluorométhyle,
R¹⁵ est chacun indépendamment un groupe halogène, un groupe acyloxy en C₂-C₈ éventuellement halogéné, un groupe alkyle en C₁-C₆ éventuellement halogéné, ou un groupe alcoxy en C₁-C₆ éventuellement halogéné,
A³ est une liaison simple ou un groupe alcanediyle en C₁-C₁₀ dans lequel une liaison éther peut intervenir dans une liaison carbone-carbone,
W¹ est un groupe hydrogène, un groupe hydrocarboné aliphatique monovalent en C₁-C₁₀ dans lequel une liaison éther, un fragment carbonyle ou un fragment carbonyloxy peut intervenir dans une liaison carbone-carbone, ou un groupe cyclique aromatique monovalent éventuellement substitué,
Rx et Ry sont chacun indépendamment un groupe hydrogène, un groupe alkyle en C₁-C₁₅ éventuellement substitué par un groupe hydroxy ou alcoxy ou un groupe cyclique aromatique monovalent éventuellement substitué, à condition que Rx et Ry ne soient pas un groupe hydrogène en même temps, Rx et Ry peuvent se lier ensemble pour former un cycle avec l'atome de carbone auquel ils sont attachés,
x³ est un entier de 0 à 2, t³ vaut 0 ou 1, f est un entier satisfaisant 0 ≤ f ≤ 5+2x³-g et g est un entier de 1 à 3.

8. Composition de réserve négative selon la revendication 7, dans laquelle le polymère comprend en outre des unités récurrentes d'au moins un type choisi parmi les unités récurrentes ayant les formules (B6), (B7) et (B8) : 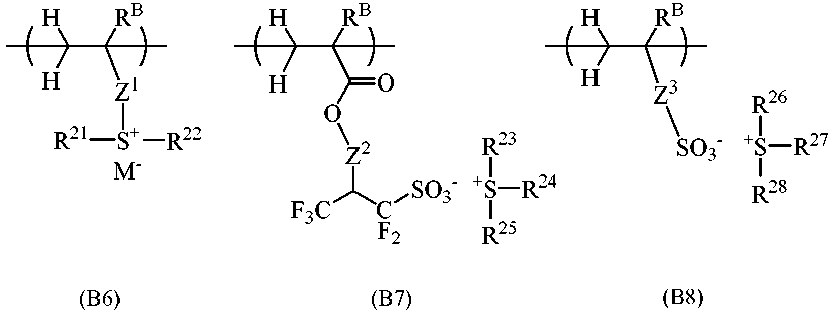
dans laquelle R^{B} est chacun indépendamment un groupe hydrogène ou méthyle,
Z¹ est une liaison simple, un groupe phénylène, O-Z¹¹-, -C(=O)-O-Z¹¹-, ou -C(=O)-NH-Z¹¹-, Z¹¹ est un groupe alcanediyle en C₁-C₆, un groupe alcèné-diyle ou phénylène en C₂-C₆, qui peut contenir un fragment carbonyle, une liaison ester, une liaison éther ou un fragment hydroxyle,
Z² est une liaison simple ou -Z²¹-C(=O)-O-, Z²¹ est un groupe hydrocarboné divalent en C₁-C₂₀ qui peut contenir un hétéroatome,
Z³ est une liaison simple, un groupe méthylène, éthylène, phénylène, phénylène fluoré, -O-Z³¹-, -C(=O)-O-Z³¹-, ou -C(=O)-NH-Z³¹-, Z³¹ est un groupe alcanediyle en C₁-C₆, alcèné-diyle ou phénylène en C₂-C₆, qui peut contenir un fragment carbonyle, une liaison ester, une liaison éther ou un fragment hydroxyle,
R²¹ à R²⁸ sont chacun indépendamment un groupe hydrocarboné monovalent en C₁-C₂₀ qui peut contenir un hétéroatome, ou R²¹ et R²² peuvent se lier ensemble pour former un cycle avec l'atome de soufre auquel ils sont attachés, ou deux ou plus parmi R²³, R²⁴ et R²⁵ peuvent se lier ensemble pour former un cycle avec l'atome de soufre auquel ils sont attachés, deux ou plus parmi R²⁶, R²⁷ et R²⁸ peuvent se lier ensemble pour former un cycle avec l'atome de soufre auquel ils sont attachés, et
M⁻ est un contre-ion non nucléophile.

9. Composition de réserve négative selon l'une quelconque des revendications 1 à 8, dans laquelle le polymère comprend des unités récurrentes ayant la formule (B 1-1), des unités récurrentes ayant la formule (B5-1) et des unités récurrentes ayant la formule (B7) : 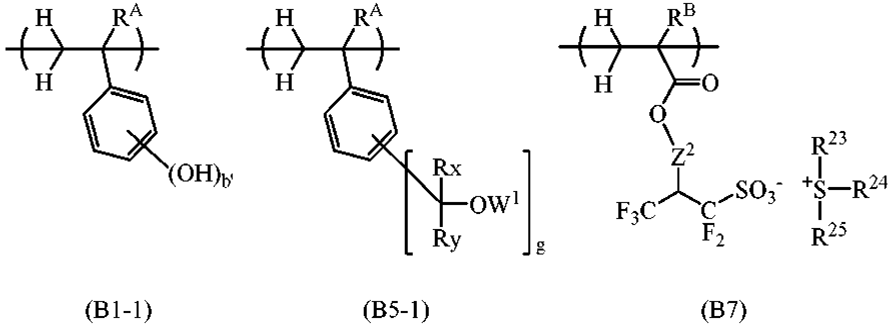
dans laquelle R^{A} est chacun indépendamment un groupe hydrogène, fluor, méthyle ou trifluorométhyle,
R^{B} est un groupe hydrogène ou méthyle,
W¹ est un groupe hydrogène, un groupe hydrocarboné aliphatique monovalent en C₁-C₁₀ dans lequel une liaison éther, un fragment carbonyle ou un fragment carbonyloxy peut intervenir dans une liaison carbone-carbone, ou un groupe cyclique aromatique monovalent éventuellement substitué,
Rx et Ry sont chacun indépendamment un groupe hydrogène, un groupe alkyle en C₁-C₁₅ éventuellement substitué par un groupe hydroxy ou alcoxy ou un groupe cyclique aromatique monovalent éventuellement substitué, à condition que Rx et Ry ne soient pas un groupe hydrogène en même temps, Rx et Ry peuvent se lier ensemble pour former un cycle avec l'atome de carbone auquel ils sont attachés,
Z² est une liaison simple ou -Z²¹-C(=O)-O-, Z²¹ est un groupe hydrocarboné divalent en C₁-C₂₀ qui peut contenir un hétéroatome,
R²³ à R²⁵ sont chacun indépendamment un groupe hydrocarboné monovalent en C₁-C₂₀ qui peut contenir un hétéroatome, ou deux ou plus parmi R²³, R²⁴ et R²⁵ peuvent se lier ensemble pour former un cycle avec l'atome de soufre auquel ils sont attachés,
b' est un entier de 1 à 3 et g est un entier de 1 à 3.

10. Composition de réserve négative selon la revendication 8 ou 9, dans laquelle le polymère de base (B) contient en outre un polymère exempt d'unités récurrentes ayant la formule (B6), d'unités récurrentes ayant la formule (B7) et d'unités récurrentes ayant la formule (B8).

11. Composition de réserve négative selon l'une quelconque des revendications 1 à 10, comprenant en outre (C) un agent de réticulation.

12. Composition de réserve négative selon la revendication 7, qui est exempte d'agent de réticulation.

13. Composition de réserve négative selon l'une quelconque des revendications 1 à 12, comprenant en outre (E) un générateur d'acide.

14. Ébauche de photomasque revêtue de la composition de réserve négative selon l'une quelconque des revendications 1 à 13.

15. Procédé de formation de motifs de réserve comprenant les étapes :
d'application de la composition de réserve négative selon l'une quelconque des revendications 1 à 13 sur un substrat pour y former un film de réserve,
d'exposition du film de réserve sous forme de motif à un rayonnement à haute énergie, et
de développement du film de réserve dans un révélateur alcalin pour former un motif de réserve.

16. Procédé selon la revendication 15, dans lequel le rayonnement à haute énergie est EUV ou EB.

17. Procédé selon la revendication 15 ou 16, dans lequel le substrat est une ébauche de photomasque.
